# EUROPÄISCHE PATENTSCHRIFT

(11) **EP 2 170 183 B1**
(45) Veröffentlichungstag und Bekanntmachung des Hinweises auf die Patenterteilung: **26.04.2017**
(21) Anmeldenummer: 08774379.5
(22) Anmeldetag: 26.06.2008
(51) Int. Cl.: A61B 17/28

(54) **CHIRURGISCHES INSTRUMENT**
SURGICAL INSTRUMENT
INSTRUMENT CHIRURGICAL

(30) Priorität: 26.06.2007 DE 102007030854
(43) Veröffentlichungstag der Anmeldung: 07.04.2010
(73) Patentinhaber: Aesculap AG, 78532 Tuttlingen (DE); Albert-Ludwigs-Universität Freiburg, 79085 Freiburg (DE)
(72) Erfinder: LUTZE, Theodor, 78582 Balgheim (DE); SCHAUER, Dirk, 10318 Berlin (DE); DISCH, Alexander, 79115 Freiburg (DE); REINECKE, Holger, 79312 Emmendingen (DE); MUELLER, Claas, 79104 Freiburg (DE)
(74) Vertreter: Hoeger, Stellrecht & Partner Patentanwälte mbB
(86) Internationale Anmeldenummer: PCT/EP2008/058205
(87) Internationale Veröffentlichungsnummer: WO 2009/000898

(56) Entgegenhaltungen:
- DE-U1- 20 020 667
- US-A- 5 318 589
- US-A- 5 486 189

## Beschreibung

Die vorliegende Erfindung betrifft chirurgisches ein Instrument mit einem Schaft, einem mindestens ein beweglich gelagertes Werkzeugelement umfassenden, eine Längsachse definierenden Endeffektor und einem im Schaft in distaler und proximaler Richtung bewegbar gelagerten Kraftübertragungsglied zum Übertragen einer an einem proximalen Ende des Instruments eingeleiteten Betätigungskraft auf den Endeffektor zum Bewegen des mindestens einen Werkzeugelements von einer ersten in eine zweite Werkzeugselementstellung und/oder umgekehrt, wobei das mindestens eine Werkzeugelement auf einer am Schaft gehaltenen Lagerwelle verschwenkbar gelagert ist, wobei das mindestens eine Werkzeugelement über mindestens ein mindestens abschnittsweise flexibles Anlenkglied mit dem Kraftübertragungsabschnitt verbunden ist zum Übertragen der Betätigungskraft vom Kraftübertragungsglied auf das mindestens eine Werkzeugelement.

Chirurgische Instrumente mit einem Schaft, einem mindestens ein beweglich gelagertes Werkzeugelement umfassenden, eine Längsachse definierenden Endeffektor und einem im Schaft in distaler und proximaler Richtung bewegbar gelagerten Kraftübertragungsglied zum Übertragen einer an einem proximalen Ende des Instruments eingeleiteten Betätigungskraft auf den Endeffektor zum Bewegen des mindestens einen Werkzeugelements von einer ersten in eine zweite Werkzeugselementstellung und/oder umgekehrt, wobei das mindestens eine Werkzeugelement auf einer am Schaft gehaltenen Lagerwelle verschwenkbar gelagert ist, werden in zahlreichen Varianten in Form von sogenannten Rohrschaftinstrumenten in der minamalinvasiven Chirurgie eingesetzt. Es ist bekannt, die verschwenkbar gelagerten Werkzeugelemente über schwenkbar am Werkzeugelement und am Kraftübertragungsglied gelagerte Lenker zu verbinden und Betätigungskräfte zum Bewegen der Werkzeugelemente zu übertragen. Eine weitere Alternative ist das Vorsehen sogenannter "Schlitz-Nocken-Führungen", wobei entweder am Werkzeugelement oder am Kraftübertragungsglied eine eine Führungsbahn definierende Ausnehmung und am jeweils anderen Teil ein in die Führungsbahn eingreifender Vorsprung ausgebildet ist, um infolge einer Relativbewegung des Kraftübertragungsglieds und des Schafts den Vorsprung in der Führungsbahn zu bewegen und so eine Bewegung des mindestens einen Werkzeugelements zu bewirken.

Nachteilig bei allen bekannten Anlenkungen der am Markt verfügbaren Instrumente sind jedoch ein infolge von unvermeidlichen Fertigungstoleranzen auftretendes Spiel sowie aufgrund der Anlenkung auftretende Reibungsverluste. Diese Nachteile führen insbesondere dazu, dass die bekannten Instrumente nicht in gewünschter Weise unter Beibehaltung ihrer Stabilität und Funktionalität miniaturisiert werden können. Schaftdurchmesser, die deutlich kleiner als 10 mm sind, sind mit herkömmlichen Kraftübertragungsmechanismen in der Praxis nicht realisierbar.

Aus der US 5,486,189 ist ein endoskopisches chirurgisches Instrument bekannt. Ein medizinisches Instrument zum Präparieren von Gewebe ist in der DE 200 20 667 U1 beschrieben. Ein weiteres chirurgisches Instrument für die endoskopische Chirurgie ist in der US 5,318,589 offenbart.

Es ist daher Aufgabe der vorliegenden Erfindung, ein chirurgisches Instrument der eingangs beschriebenen Art so zu verbessern, dass der Endeffektor besonders spiel- und reibungsarm wird.

Diese Aufgabe wird bei einem chirurgischen Instrument der eingangs beschriebenen Art erfindungsgemäß dadurch gelöst, dass das mindestens eine Anlenkglied unlösbar mit dem mindestens einen Werkzeugelement verbunden ist.

Das Anlenkglied, das selbstverständlich auch vollständig, das heißt über seine gesamte Länge, flexibel ausgebildet sein kann, gestattet eine für eine Bewegung des mindestens einen Werkzeugelements erforderliche Verformung, so dass auf bekannte Anlenkungsvarianten, wie zum Beispiel "Schlitz-Nocken-Führungen" oder schwenkbare Anlenkungen von kleinen Lenkern zwischen dem mindestens einen Werkzeugelement und dem Kraftübertragungsglied, verzichtet werden kann. Durch das Vorsehen mindestens eines Anlenkglieds in der beschriebenen Art und Weise werden somit im Vergleich zu den aus dem Stand der Technik bekannten Lösungen die Reibungsverluste minimiert. Zu dem ist die erfindungsgemäß vorgeschlagene Anlenkung des mindestens einen Werkzeugelements äußerst spielarm, je nach Ausgestaltung sogar praktisch spielfrei. Da auf komplizierte Anlenkungen zwischen dem Kraftübertragungsglied und dem mindestens einen Werkzeugelement verzichtet werden kann, ist es mit einem solchen Anlenkglied möglich, eine Größe des Endeffektors deutlich zu verringern, das heißt insbesondere Durchmesser von Endeffektoren zu realisieren, die auch kleiner als 2 mm sein können. Damit eignet sich das erfindungsgemäß vorgeschlagene Instrument hervorragend für die Verwendung bei neurochirurgischen Eingriffen oder minimalinvasiven Eingriffen bei Kindern. Besonders spiel- und reibungsarm wird der Endeffektor dadurch, dass das mindestens eine Anlenkglied unlösbar mit dem mindestens einen Werkzeugelement verbunden ist. Ferner kann so auch verhindert werden, dass sich Teile bei besonders kleinen Endeffektoren lösen und im Körper eines Patienten verloren gehen können.

Besonders einfach hergestellt und mit ausreichender Stabilität ausgestattet werden kann ein chirurgisches Instrument, wenn das mindestens eine Anlenkglied einstückig mit dem mindestens einen Werkzeugelement ausgebildet ist.

Vorzugsweise ist das mindestens eine Anlenkglied unlösbar mit dem Kraftübertragungsglied verbunden. So können auch im Bereich der Kraftübertragung vom Kraftübertragungsglied auf das Anlenkglied Reibungsverluste minimiert und die Anlenkung besonders spielfrei ausgebildet werden.

Die Stabilität des Endeffektors kann weiter erhöht werden, wenn das mindestens eine Anlenkglied einstückig mit dem Kraftübertragungsglied ausgebildet ist.

Insbesondere ist es vorteilhaft, wenn das mindestens eine Anlenkglied ein Festkörpergelenk zwischen dem Kraftübertragungsglied und dem mindestens einen Werkzeugelement bildet. Durch das Vorsehen eines Festkörpergelenks kann auf jede andere Form von ineinander greifenden Teilen verzichtet werden, um eine Kraft vom Kraftübertragungsglied auf das mindestens eine Werkzeugelement zu übertragen. Festkörpergelenke ermöglichen somit eine praktisch spielfreie Kraftübertragung und weisen auch bei einer maximalen Miniaturisierung noch eine ausreichende Stabilität für eine gewünschte Funktion des Instruments auf.

Um das mindestens eine Werkzeugelement insbesondere durch Übertragen einer Zugkraft von der Längsachse weg schwenken zu können, ist es günstig, wenn das mindestens eine Anlenkglied auf einer von der Längsachse weg weisenden Außenseite am mindestens einen Werkzeugelement angreift.

Günstig kann es jedoch auch sein, wenn das mindestens eine Anlenkglied auf einer auf die Längsachse hinweisende Innenseite oder eines inneren Abschnitts am mindestens einen Werkzeugselement angreift. Diese Ausgestaltung ermöglicht es, das mindestens eine Werkzeugelement insbesondere durch Beaufschlagen des Anlenkglieds mit einer Zugkraft in Richtung auf die Längsachse hin zu bewegen. Bei zwei vorgesehenen Werkzeugelementen können diese so infolge einer ausgeübten Zugkraft geschlossen werden.

Eine Stabilität des Instruments insgesamt kann weiter dadurch erhöht werden, dass ein proximales Ende des Endeffektors fest mit dem Kraftübertragungsglied verbunden ist.

Vorzugsweise ist ein proximales Ende des Endeffektors einstückig mit dem Kraftübertragungsglied ausgebildet. Dadurch kann in der Herstellung auf eine möglicherweise aufwändige Verbindung zwischen dem Endeffektor und dem Kraftübertragungsglied verzichtet werden, die zudem eine Stabilität des Instruments limitieren kann.

Gemäß einer bevorzugten Ausführungsform der Erfindung kann vorgesehen sein, dass der Endeffektor einen in distaler und proximaler Richtung bewegbaren Kraftübertragungsabschnitt umfasst, welcher mit dem Kraftübertragungsglied verbunden ist, oder an diesem anliegt, und einen distalen Endbereich des Kraftübertragungsglieds bildet, und dass ein proximales Ende des mindestens einen Anlenkglieds mit dem Kraftübertragungsabschnitt verbunden ist. Durch das Vorsehen des Kraftübertragungsabschnitts kann der Endeffektor als eigenständige Einheit ausgebildet werden, insbesondere auch einstückig. Dies hat fertigungstechnisch Vorteile, insbesondere dann, wenn der Endeffektor sehr stark miniaturisiert werden soll. Das Kraftübertragungsglied und der Endeffektor können so getrennt gefertigt werden. Die Kraftübertragung auf das mindestens eine Werkzeugelement erfolgt dann ausgehend vom Kraftübertragungsabschnitt über das Anlenkglied auf das zu bewegende Werkzeugelement.

Ein besonders kompakter Aufbau des Endeffektors lässt sich insbesondere dadurch erreichen, dass das mindestens eine Anlenkglied einstückig mit dem Kraftübertragungsabschnitt ausgebildet ist.

Einer der oben beschriebenen Endeffektoren lässt sich insbesondere auf einfache Weise in Verbindung mit herkömmlichen Instrumenten einsetzen, wenn das Kraftübertragungsglied eine Schub- und Zugstange umfasst. Insbesondere kann das Kraftübertragungsglied selbst als Schub- und Zugstange ausgebildet sein.

Vorteilhaft ist es ferner, wenn das Kraftübertragungsglied mindestens einen im Schaft verschiebbar gelagerten Kolben und einen vom mindestens einen Kolben durch ein Dichtungselement getrennten Fluidabschnitt umfasst, welcher mit einem Fluid befüllt ist, welches durch Druckbeaufschlagung gegen das Dichtungselement drückbar ist zum Bewegen des am Dichtungselement anliegenden mindestens einen Kolbens parallel zur Längsachse des Schafts. Einen derartigen Fluidabschnitt vorzusehen, ermöglicht es, einen Schaft flexibel oder gekrümmt auszubilden und trotzdem eine gewünschte Kraftübertragung ausgehend von einem proximalen Ende des Instruments zum mindestens einen Werkzeugelement sicherzustellen. Zudem ist eine fluidische Kraftübertragung besonders spielarm, in der Regel sogar praktisch spielfrei, und nahezu nicht mit Reibungsverlusten behaftet.

Das Instrument lässt sich besonders kompakt ausbilden, wenn der mindestens eine Kolben den Kraftübertragungsabschnitt umfasst. Insbesondere kann der Kolben den Kraftübertragungsabschnitt bilden.

Um eine geschlossene fluidische Kraftübertragung ausbilden zu können, ist es günstig, wenn das Kraftübertragungsglied zwei Kolben umfasst, welche zwischen sich den von den Kolben durch jeweils ein Dichtungselement getrennten Fluidabschnitt distalseitig und proximalseitig begrenzen.

Um Verluste eines in den Fluidabschnitt eingefüllten Fluids, bei dem es sich vorzugsweise um eine körperverträgliche Flüssigkeit wie zum Beispiel eine physiologische Kochsalzlösung handelt, zu verhindern, ist es günstig, wenn der Fluidabschnitt einen Fluidraum umfasst, welcher vollständig geschlossen und abgedichtet ist.

Eine optimale Abdichtung des Fluidabschnitts mit dem Dichtungselement kann insbesondere dadurch erreicht werden, dass das Dichtungselement flexibel und inelastisch ist. So wird verhindert, dass infolge einer Druckerhöhung im Fluid eine Hülle des Fluidabschnitts bersten kann.

Einfach in der Herstellung, auf einfache Weise mit einem Schaft verbindbar und zudem lange Verschiebewege gestattbar kann ein Dichtungselement sein, wenn es in Form eines Balgs oder eines Rollbalgs ausgebildet ist.

Um zu verhindern, dass sich der Kraftübertragungsabschnitt und/oder das Kraftübertragungsglied relativ zum Schaft um dessen Längsachse verdrehen können, ist es vorteilhaft, wenn das Instrument eine Führungseinrichtung zum Führen einer Bewegung des Kraftübertragungsabschnitts und/oder des Kraftübertragungsglieds im Schaft parallel zur Längsachse aufweist.

Günstigerweise bildet die Führungseinrichtung eine Verdrehsicherung zum Verhindern einer Verdrehung des mindestens einen Kolbens um die Längsachse. Damit kann insbesondere ein Verklemmen des Endeffektors im Schaft beziehungsweise des mindestens einen Werkzeugelements auf der Lagerwelle sicher verhindert werden.

Besonders einfach wird der Aufbau der Führungseinrichtung, wenn diese erste und zweite zusammenwirkende Führungsglieder umfasst, von denen eines am Kraftübertragungsabschnitt und ein anderes am Schaft angeordnet ist, und wenn das erste Führungsglied in Form eines Führungsvorsprungs und das zweite Führungsglied in Form einer Führungsausnehmung ausgebildet ist.

Eine Längsführung lässt sich auf einfache Weise dadurch ausbilden, dass die Führungsausnehmung in Form einer sich parallel zur Längsachse erstreckenden Führungsnut ausgebildet ist. In dieser Führungsnut kann sich insbesondere ein korrespondierender Führungsvorsprung parallel zur Längsachse bewegen.

Damit das Kraftübertragungsglied für eine Bedienperson auf einfache und sichere Weise parallel zur Längsachse, vorzugsweise in distaler und proximaler Richtung, bewegt werden kann, ist günstigerweise eine Betätigungseinrichtung am proximalen Ende des Instruments zum Bewegen des Kraftübertragungsglieds parallel zur Längsachse vorgesehen.

Eine Handhabung des Instruments kann bevorzugt dadurch verbessert werden, dass die Betätigungseinrichtung mindestens ein bewegbares Griffelement umfasst, welches mit dem Kraftübertragungsglied gelenkig verbunden ist.

Unter einer gelenkigen Verbindung ist insbesondere auch zu verstehen, dass das Griffelement an einem proximalen Ende des Kraftübertragungsglieds anliegt und dieses in distaler Richtung bewegen kann.

Damit das Instrument auf einfache Weise gereinigt werden kann, ist es vorteilhaft, wenn die Betätigungseinrichtung mit dem Schaft und/oder dem Kraftübertragungsglied lösbar verbindbar ist. Zudem wäre es so auch denkbar, den Schaft mit dem Endeffektor als Einheit auszubilden, welche als Einwegprodukt zur einmaligen Verwendung mit einer mehrfach verwendbaren Betätigungseinrichtung genutzt werden kann.

Damit ein Operateur eine Orientierung des Endeffektors, insbesondere des mindestens einen Werkzeugelements, direkt an einer Stellung der Betätigungseinrichtung erkennen kann, ist es günstig, wenn der Schaft relativ zur Betätigungseinrichtung feststehend angeordnet ist.

Um es einem Operateur zu ermöglichen, das Instrument bequem in einer ihm angenehmen Stellung zu halten und das mindestens eine Werkzeugelement trotzdem im Inneren eines Patientenkörpers zur Durchführung eines chirurgischen Eingriffs in erforderlicher Weise einstellen zu können, ist es vorteilhaft, wenn der Schaft und/oder das Kraftübertragungsglied relativ zur Betätigungseinrichtung um die Längsachse verdrehbar sind.

Günstig ist es, wenn das mindestens eine Anlenkglied in einer Werkzeugelementstellung, welche eine Grundstellung definiert, unverformt ist. Insbesondere kann ein Instrument mit zwei Werkzeugelementen so ausgebildet sein, dass die beiden Werkzeugelemente in der Grundstellung geschlossen oder aufgespreizt sind. So kann in der Grundstellung das mindestens eine Anlenkglied keine Kräfte auf das mindestens eine Werkzeugelement oder das Kraftübertragungsglied ausüben.

Gemäß einer weiteren bevorzugten Ausführungsform der Erfindung kann vorgesehen sein, dass das mindestens eine Anlenkglied in einer beliebigen von einer Grundstellung, in welcher es unverformt ist, abweichenden Arbeitsstellung des mindestens einen Werkzeugelements verformt ist. Bei der Arbeitsstellung handelt es sich ebenfalls um eine Werkzeugelementstellung, die jedoch nicht mit der Grundstellung übereinstimmt. Diese Ausgestaltung des Instruments hat den Vorteil, dass durch Verformung des Anlenkglieds Energie gespeichert werden kann, um das mindestens eine Werkzeugelement ganz oder teilweise von der ausgelenkten Arbeitsstellung wieder in die Grundstellung zurückzuüberführen. Das Anlenkglied kann so gleichzeitig als Rückstellglied einer Rückstelleinrichtung dienen.

Um einen möglichst schlanken und kompakten Aufbau des Instruments, insbesondere dessen Endeffektors, zu ermöglichen, ist es günstig, wenn sich das mindestens eine Anlenkglied parallel oder im Wesentlichen parallel zur Längsachse sowohl proximalseitig als auch distalseitig der Lagerwelle erstreckt. Ferner kann durch eine entsprechende Länge des Anlenkglieds auch ein gewünschter Öffnungswinkel oder Verschwenkwinkel des mindestens einen Werkzeugelements erreicht werden. Eine Verschenkbewegung des mindestens einen Werkzeugelements lässt sich optional auch über eine entsprechende Begrenzung an der Betätigungseinrichtung oder durch entsprechende Begrenzung eines Verschiebewegs des Kraftübertragungsglieds im Schaft einstellen.

Der Endeffektor kann besonders kompakt aufgebaut werden, wenn sich das mindestens eine Anlenkglied proximalseitig und distalseitig der Lagerwelle jeweils gleich oder in etwa gleich weit erstreckt. So können insbesondere alle erforderlichen Teile für die Anlenkung des mindestens einen Werkzeugelements in Richtung der Längsachse hintereinander angeordnet werden, wodurch sich ein Außendurchmesser des Schafts beziehungsweise ein Außendurchmesser des Endeffektors deutlich verringern lassen.

Besonders einfach wird der Aufbau des Endeffektors, wenn das mindestens eine Anlenkglied in Form eines dünnen Steges ausgebildet ist. Insbesondere kann eine Dicke des vorzugsweise streifenförmigen Steges sehr viel kleiner sein als eine Breite desselben. Hier können Verhältnisse von 1:10 oder sogar kleiner als 1:20 vorgesehen sein.

Um einen besonders kleinen Endeffektor auszubilden, ist es günstig, wenn das Anlenkglied, insbesondere wenn es in Form eines Steges ausgebildet ist, eine Dicke in einem Bereich von etwa 50 µm bis 700 µm aufweist. Vorzugsweise beträgt die Dicke 100 µm bis 200 µm.

Damit das mindestens eine Werkzeugelement, welches aus einer Grundstellung ausgelenkt ist, wieder selbsttätig in diese zurücküberführt werden kann, wenn keine Betätigungskraft auf das Kraftübertragungsglied ausgeübt wird, ist es vorteilhaft, wenn eine Rückstelleinrichtung vorgesehen ist zum Überführen des mindestens einen Werkzeugelements aus einer beliebigen ausgelenkten Werkzeugelementstellung zurück in eine Grundstellung.

Der Aufbau der Rückstelleinrichtung wird besonders einfach, wenn diese mindestens ein Rückstellglied umfasst, welches sich einerseits am mindestens einen Werkzeugelement und andererseits am Kraftübertragungsglied abstützt. Insbesondere kann sich das Rückstellglied auch am Kraftübertragungsabschnitt des Endeffektors abstützen, wenn dieser beispielsweise ein distales Ende des Kraftübertragungsglieds bildet.

Eine Rückstellkraft kann insbesondere auf einfache Weise durch das mindestens eine Rückstellglied dadurch ausgeübt werden, wenn es federelastisch ausgebildet ist. Infolge einer Verformung des Rückstellglieds gespeicherte Energie kann dann wiederum zum Überführen des mindestens einen Werkzeugelements von einer ausgelenkten Werkzeugelementstellung in die Grundstellung zurück genutzt werden.

Der Aufbau des Instruments, insbesondere des Endeffektors, kann besonders kompakt gestaltet werden, wenn das mindestens eine Anlenkglied das Rückstellglied bildet. Das Anlenkglied übt somit eine Doppelfunktion aus. Auf ein separates Rückstellglied kann in diesem Falle vollständig verzichtet werden.

Der Aufbau des Instruments vereinfacht sich und damit insbesondere auch dessen Herstellung, wenn das distale Ende des Instruments spiegelsymmetrisch zu mindestens einer Symmetrieebene ausgebildet ist. So kann insbesondere auch bei Vorsehen von zwei oder mehr Werkzeugelementen eine symmetrische Betätigung derselben bereits durch den Aufbau des Endeffektors vorgegeben werden.

Der Endeffektor lässt sich besonders einfach ausbilden und herstellen, wenn der Kraftübertragungsabschnitt zweiteilig ausgebildet ist und zwei im Wesentlichen durch eine die Längsachse enthaltende Ebene getrennte Abschnittshälften umfasst. Beispielsweise kann ein insgesamt im Wesentlichen zylindrisch geformter Kraftübertragungsabschnitt durch zwei Zylinderhälften ausgebildet werden.

Das Instrument lässt sich auf einfache Weise in Form einer Schere, einer Pinzette oder einer Fasszange ausbilden, wenn es zwei Werkzeugelemente umfasst.

Die Herstellung des Endeffektors kann insbesondere dadurch vereinfacht werden, wenn er zweiteilig ausgebildet ist und zwei Endeffektorteile umfasst. Damit lassen sich insbesondere komplizierte Formgebungen an den Werkzeugelementen einfacher realisieren, beispielsweise durch Fräsen, Draht- oder Lasererodieren.

Zur Ausbildung eines symmetrischen oder im Wesentlichen symmetrischen Endeffektors ist es günstig, wenn jeder Endeffektorteil ein Werkzeugelement umfasst.

Des Weiteren kann die Herstellung vereinfacht und die Stabilität jedes Endeffektorteils erhöht werden, wenn jeder Endeffektorteil eine Abschnittshälfte des Kraftübertragungsabschnitts umfasst.

Damit nur eine Art oder ein Typ von Endeffektorteilen hergestellt werden muss, um einen zweiteiligen Endeffektor auszubilden, ist es günstig, wenn der Endeffektor aus zwei identischen, fest miteinander verbundenen Endeffektorteilen gebildet ist, welche relativ zueinander um 180° um die Längsachse verdreht angeordnet sind. Zwei identische Effektorteile lassen sich dann so zusammensetzen, dass sie einen einzigen Endeffektor ausbilden.

Gemäß einer weiteren bevorzugten Ausführungsform der Erfindung kann vorgesehen sein, dass die zwei Werkzeugelemente einander gegenseitig durchdringend oder kreuzend ausgebildet sind. Darunter ist insbesondere zu verstehen, dass an einem Werkzeugelement eine Durchbrechung oder Ausnehmung vorgesehen sein kann, durch welche das andere Werkzeugelement, beispielsweise ein distales Ende desselben, durch- oder eingeführt ist. Eine durchdringende oder kreuzende Ausbildung der Werkzeugelemente ermöglicht insbesondere eine Verschwenkung derselben aufeinander zu durch Beaufschlagen des Anlenkglieds mit einer Zugkraft. So können insbesondere Scheren, Fasszangen und Pinzetten ausgebildet werden, die in einer Grundstellung geöffnet sind und durch Ausüben einer Zugkraft auf das Kraftübertragungsglied geschlossen werden können.

Vorzugsweise ist eine Verbindungseinrichtung vorgesehen zum Verbinden der Endeffektorteile miteinander. Die Endeffektorteile können so separat hergestellt, miteinander verbunden und dann in den Schaft des Instruments eingesetzt werden. Selbstverständlich können sie optional auch miteinander verklebt oder verschweißt werden.

Besonders einfach wird der Aufbau des Endeffektors und damit auch des Instruments, wenn die Verbindungseinrichtung mindestens ein erstes und mindestens ein zweites Verbindungselement umfasst, welche in einer Verbindungsstellung miteinander in Eingriff stehen, und wenn das mindestens eine erste und das mindestens eine zweite Verbindungselement jeweils an einem Endeffektorteil angeordnet sind. Zwei Endeffektorteile können so auf einfache Weise dadurch miteinander verbunden werden, dass die mindestens einen ersten und zweiten Verbindungselemente miteinander in Eingriff gebracht werden, so dass sie die Verbindungsstellung einnehmen.

Der Aufbau der Verbindungseinrichtung wird besonders einfach, wenn das mindestens eine erste Verbindungselement in Form eines Vorsprungs und wenn das mindestens eine zweite Verbindungselement in Form einer zum Vorsprung korrespondierenden Aufnahme ausgebildet sind. Vorsprünge und Ausnehmungen lassen sich besonders einfach herstellen. Zudem können sie auch als gegenseitige Anschläge dienen, um eine Relativposition der miteinander zu verbindenden Endeffektorteile in gewünschter Weise vorgeben zu können.

Vorzugsweise sind das mindestens eine erste und das mindestens eine zweite Verbindungselement in einer Richtung quer oder im Wesentlichen quer zur Längsachse weisend orientiert. Beispielsweise können sie vom einen Endeffektorteil in Richtung auf das andere Endeffektorteil hin weisend abstehend ausgebildet sein, und zwar quer zur Längsachse. Dies verhindert, dass infolge einer Bewegung des Kraftübertragungsglieds parallel zur Längsachse die Endeffektorteile auseinander bewegt werden können.

Eine Verbindung von zwei Endeffektorteilen wird besonders einfach, wenn sich das mindestens eine erste und das mindestens eine zweite Verbindungselement in einer Richtung parallel oder im Wesentlichen parallel zur Längsachse erstrecken. Beispielsweise können die Verbindungselemente in Form von Nuten und formschlüssig in diese eingreifende Vorsprünge ausgebildet sein, die zwar quer zur Längsachse von jeweils einem Endeffektorteil weg weisen, sich jedoch in einer Richtung parallel zur Längsachse erstrecken. Alternativ wäre es auch denkbar, dass eine Nut quer zur Längsachse verläuft und ein Verbindungselement bildet. Die ineinander greifenden Verbindungselemente können insbesondere so ausgebildet sein, dass sie klemmend und/oder rastend miteinander in der Verbindungsstellung in Eingriff stehen. Optional kann eine, insbesondere auch begrenzte, relative Bewegbarkeit der beiden Endeffektorteile in der Verbindungsstellung zu Justagezwecken wünschenswert sein.

Um den Endeffektor besonders kompakt aufbauen zu können, ist es günstig, wenn die Verbindungseinrichtung am Kraftübertragungsabschnitt angeordnet ist zum Verbinden der Abschnittshälften. Damit stört die Verbindungseinrichtung räumlich das mindestens eine Werkzeugelement, insbesondere eine Bewegung desselben, nicht.

Gemäß einer bevorzugten Ausführungsform der Erfindung kann vorgesehen sein, dass der Endeffektor derart ausgebildet ist, dass das mindestens eine Werkzeugelement infolge einer Bewegung des Kraftübertragungsglieds in proximaler Richtung in Richtung auf die Längsachse hin verschwenkbar ist und umgekehrt. Mit einem solchen Endeffektor lässt sich beispielsweise eine Fasszange ausbilden, welche auf Zug schließt.

Vorteilhaft ist es ferner, wenn der Endeffektor derart ausgebildet ist, dass das mindestens eine Werkzeugelement infolge einer Bewegung des Kraftübertragungsglieds in distaler Richtung in Richtung auf die Längsachse hin verschwenkbar ist und umgekehrt. Beispielsweise lässt sich so ein chirurgisches Instrument, beispielsweise eine Schere oder eine Fasszange ausbilden, die infolge einer in distaler Richtung wirkenden Druckbeaufschlagung des Kraftübertragungsglieds geschlossen werden können.

Günstig kann es ferner sein, wenn der Endeffektor zwei Werkzeugelemente umfasst und derart ausgebildet ist, dass distale Enden der Werkzeugelemente infolge einer mit dem Kraftübertragungsglied übertragbaren Schubkraft aufeinander zu verschwenkbar sind. So kann beispielsweise mit einer Fasszange Gewebe gegriffen und gehalten werden, indem das Kraftübertragungsglied in distaler Richtung mit einer Schubkraft beaufschlagt wird.

Insbesondere zur Ausbildung einer Schere ist es vorteilhaft, wenn der Endeffektor zwei Werkzeugelemente umfasst und derart ausgebildet ist, dass distale Enden der Werkzeugelemente infolge einer mit dem Kraftübertragungsglied übertragbaren Zugkraft aufeinander zu verschwenkbar sind. Eine solche Ausgestaltung kann sich insbesondere auch zur Ausbildung einer Fasszange hervorragend eignen.

Um das mindestens eine Werkzeugelement in definierter Weise auf der Lagerwelle lagern zu können, ist es vorteilhaft, wenn ein proximales Ende des mindestens einen Werkzeugelements in Form mindestens eines Lagerbackens ausgebildet ist, welcher eine zur Lagerwelle korrespondierend ausgebildete Lagerwellenaufnahme aufweist.

Besonders einfach in der Herstellung und im Aufbau wird der Endeffektor, wenn der mindestens eine Lagerbacken im Wesentlichen quader- oder würfelförmig ausgebildet ist. Vorzugsweise ist der mindestens eine Lagerbacken vom Kraftübertragungsglied beabstandet. Der Abstand entspricht mindestens einer Erstreckung des Lagerbackens parallel zur Längsachse, kann jedoch auch zwei- oder dreimal so groß sein.

Vorzugsweise weist der mindestens eine Lagerbacken eine Außenfläche auf, welche an eine Innenkontur des Schafts angepasst ist. Bei einem langgestreckten hülsenförmigen oder rohrförmigen Schaft ist die Außenfläche des Lagerbackens vorzugsweise in Form eines Kugeloberflächenabschnitts ausgebildet. Dies ermöglicht eine Verschwenkung des Werkzeugelements auch dann, wenn die Außenfläche des mindestens einen Lagerbackens direkt in einer Innenwand des rohrförmigen Schafts anliegt.

Vorzugsweise ist eine Breite des mindestens einen Lagerbackens in einer Richtung parallel zu einer von der Lagerwelle definierten Schwenkachse etwa halb so groß wie ein Innendurchmesser des Schafts. Dies gestattet es, zwei Lagerbacken nebeneinander auf der Lagerwelle zu lagern, beispielsweise um einen Endeffektor mit zwei relativ zueinander verschwenkbaren Werkzeugelementen auszubilden.

Vorteilhaft ist es, wenn an einem proximalen Ende des mindestens einen Werkzeugelements ein Anlenkgliedstützabschnitt ausgebildet ist, welcher in Richtung auf das mindestens eine Anlenkglied hin weist. Vorzugsweise ist der Anlenkgliedstützabschnitt eben und flächenhaft ausgebildet. Er ermöglicht es insbesondere bei einer sogenannten Überkreuzanordnung von zwei Festkörpergelenken sowie der zugehörigen Werkzeugelemente, also bei gekreuzten Werkzeugelementen, eine Stabilisierung der Festkörpergelenke bei Greifvorgängen, bei denen hohe Kräfte über die Festkörpergelenke übertragen werden. Dieser Fall tritt insbesondere dann auf, wenn die Werkzeugelemente auf Zug belastet werden. Insbesondere bei gekreuzten Werkzeugelementen können diese zum Öffnen auf Druck, zum Schließen jedoch auf Zug belastet werden. Die Belastung auf Zug verhindert im Gegensatz zu der Belastung auf Schub oder Druck ein Nachgeben, das sogenannte "Buckling" der biegelastischen Festkörpergelenke. Allerdings kann es durch die Beanspruchung der Festkörpergelenke auf Zug im belasteten Zustand der Werkzeugelemente, also insbesondere bei Greifvorgängen, zu einem sogenannten "Straffziehen" der Festkörpergelenke kommen. Die negative Folge hiervon sind hohe Kerbspannungen in den Anfangs- und Endbereichen der Festkörpergelenke, also insbesondere in den Anlenkgliedern. Des Weiteren vermindert sich durch das "Straffziehen" die Steifigkeit des Endeffektors im belasteten Zustand. Der nachteilige Effekt des "Straffziehens" nimmt mit größer werdenden Öffnungswinkeln der Werkzeugelemente zu. Die Anlenkgliedstützabschnitte wirken diesen Nachteilen entgegen, insbesondere helfen sie das "Straffziehen" der Festkörpergelenke im belasteten Zustand zu reduzieren und eine natürliche Biegelinie der Festkörpergelenke beziehungsweise Anlenkglieder zu unterstützen. Dies kann insbesondere dadurch erreicht werden, dass die Anlenkgliedstützabschnitte in einer entsprechenden Stellung der Werkzeugelemente flächig an den Anlenkgliedern anliegen. Insbesondere bei Greifvorgängen gekreuzter Werkzeugelemente mit hohen Kräften und großen Öffnungswinkeln ergeben sich so deutliche Vorteile gegenüber nicht abgestützten Anlenkgliedern.

Ein besonders einfacher Aufbau des chirurgischen Instruments kann dadurch erreicht werden, dass die mindestens eine Lagerbacke den Anlenkgliedstützabschnitt umfasst. Der Anlenkgliedstützabschnitt kann so insbesondere in die Lagerbacke integriert werden, weitere Elemente oder Bauteile am Instrument sind dann nicht erforderlich.

Um die Vorteile der mindestens abschnittsweise flexiblen Anlenkglieder noch besser nutzen zu können, ist es günstig, wenn das mindestens eine Anlenkglied in einer Werkzeugelementstellung, welche eine Grundstellung definiert, unverformt und vom Anlenkgliedstützabschnitt beabstandet ist. Insbesondere bei gekreuzten Werkzeugelementen können diese so auf Schub oder Druck geöffnet werden. Zu einer Abstützung der Anlenkglieder durch den Anlenkgliedstützabschnitt kann es insbesondere dann kommen, wenn mit den Werkzeugelementen ein Gegenstand oder Gewebe gegriffen und dadurch eine Bewegung derselben aufeinander zu blockiert wird. Erst dann kann es optional möglich sein, dass der Anlenkgliedstützabschnitt in Kontakt mit dem mindestens einen Anlenkglied kommt, um dieses in der beschriebenen Weise abzustützen, was eine vorteilhafte Reduktion von Kerbspannungen an dem mindestens einen Anlenkglied zur Folge hat sowie zu einer Verringerung von Bauteilbelastungen im belasteten Zustand der Werkzeugelemente, also insbesondere bei Greifvorgängen unter großen Öffnungswinkeln, führt.

Gemäß einer bevorzugten Ausführungsform der Erfindung kann vorgesehen sein, dass sich in einer Werkzeugelementstellung, welche eine Zugstellung definiert, der Anlenkgliedstützabschnitt flächig am mindestens einen Anlenkglied abstützt, wobei in der Zugstellung das mindestens eine Werkzeugelement ausgelenkt und in der ausgelenkten Stellung blockiert ist. Insbesondere kann der Anlenkgliedstützabschnitt so angeordnet und ausgebildet sein, dass es nur dann, wenn die Werkzeugelemente die Zugstellung definieren oder einnehmen, zu einer flächigen Abstützung des Anlenkglieds mittels des Anlenkgliedstützabschnitts kommt. Der Abstützungseffekt kann also, abhängig von einer jeweiligen Belastungssituation der Werkzeugelemente, bereits bei Öffnungswinkeln der Werkzeugelemente unterhalb eines vorgegebenen Zielöffnungswinkels eintreten. Mit anderen Worten bedeutet dies, dass der gewünschte beschriebene Abstützungseffekt an den Festkörpergelenken, insbesondere bei Greifvorgängen unter großen Öffnungswinkeln der Werkzeugelemente, eintreten kann.

Günstigerweise ist in der Zugstellung das mindestens eine Werkzeugelement um mindestens 30° gegenüber einer Grundstellung, in welcher das mindestens eine Anlenkglied unverformt ist, ausgelenkt. Diese Ausgestaltung ermöglicht es, den beschriebenen Abstützungseffekt mittels des Anlenkgliedstützabschnitts zu erreichen. Unterhalb eines Auslenkwinkels von 20° kommt es daher nicht zu einem Kontakt zwischen dem mindestens einen Anlenkglied und dem Anlenkgliedstützabschnitt. Bei zwei relativ zueinander verschwenkbaren Werkzeugelementen entspricht eine Auslenkung von 20° eines Werkzeugelements bezogen auf die Grundstellung insgesamt einem Öffnungswinkel zwischen den Werkzeugelementen von 40°.

Gemäß einer weiteren bevorzugten Ausführungsform der Erfindung kann vorgesehen sein, dass ein erstes Werkzeugelement zwei erste Lagerbacken aufweist, welche quer zur Längsachse voneinander beabstandet angeordnet sind, und dass ein zweites Werkzeugelement einen zweiten Lagerbacken aufweist, welcher zwischen den zwei ersten Lagerbacken auf der Lagerwelle gelagert ist. So lässt sich insgesamt eine spiegelsymmetrische Anordnung von zwei Werkzeugelementen erreichen, bei denen insbesondere das zweite Werkzeugelement das erste Werkzeugelement durchdringend oder in eine Ausnehmung desselben eingreifend auf der Lagerwelle gelagert werden kann. Hierzu weist das erste Werkzeugelement optional eine Durchbrechung oder einen entsprechenden Schlitz für das zweite Werkzeugelement oder ein Werkzeugende desselben auf.

Vorteilhafterweise weist das mindestens eine Werkzeugelement ein Werkzeugende in Form einer Schneide oder in Form eines Klemmbackens auf. Damit lassen sich Endeffektoren in Form von Schneid-, Klemm- oder Fasseinrichtungen ausbilden.

Um ein besonders kleines Instrument ausbilden zu können ist es günstig, wenn das Werkzeugende eine Materialstärke in einem Bereich von 300 µm bis 700 µm aufweist. Insbesondere kann die Materialstärke die Dicke einer Schneide definieren.

Vorzugsweise erstreckt sich das Werkzeugende distalseitig von einem Verbindungsbereich zwischen dem mindestens einen Anlenkglied und dem mindestens einen Werkzeugelement. So kann das mindestens eine Werkzeugelement frei von für eine Funktion des Instruments hinderlichen Teilen gehalten werden.

Körpergewebe lässt sich insbesondere auf einfache Weise dadurch schneiden, greifen beziehungsweise halten, wenn das chirurgische Instrument in Form einer Schere, einer Fasszange oder einer Pinzette ausgebildet ist.

Grundsätzlich ist es denkbar, das chirurgische Instrument aus beliebigen, zur Herstellung von chirurgischen Instrumenten geeigneten Materialien herzustellen. Der Endeffektor wird vorzugsweise jedoch aus einem Metall oder einem Kunststoff hergestellt. Die Herstellung aus einem Kunststoff, beispielsweise Polyetheretherketon (PEEK), ermöglicht grundsätzlich eine Herstellung des Endeffektors durch ein Thermoformungsverfahren, wie beispielsweise Spritzgießen. Der Endeffektor, der aus einem Metall hergestellt ist, beispielsweise einem Instrumentenstahl, kann besonders stabil und damit besonders klein ausgebildet werden.

Vorzugsweise liegt ein Außendurchmesser des Schafts in einem Bereich von 1,5 mm bis 8 mm.

Günstig ist es, wenn er in einem Bereich von 1,5 mm bis 4 mm liegt. Vorzugsweise ist er kleiner als 3 mm. Insbesondere mit den kleinsten genannten Schaftaußendurchmessern können minimalinvasive chirurgische Eingriffe am Kopf oder bei kleinen Kindern unter Vermeidung größerer Traumata vorgenommen werden.

Die nachfolgende Beschreibung bevorzugter Ausführungsformen der Erfindung dient im Zusammenhang mit der Zeichnung der näheren Erläuterung. Es zeigen:
- Figur 1:: eine teilweise durchbrochene perspektivische Gesamtansicht eines chirurgischen Instruments;
- Figur 2:: eine perspektivische vergrößerte Ansicht des Bereichs A aus Figur 1;
- Figur 3:: eine perspektivische Darstellung eines Endeffektors des in den Figuren 1 und 2 dargestellten Instruments in einer Verbindungsstellung;
- Figur 4:: eine Explosionsdarstellung des Endeffektors aus Figur 3;
- Figur 5:: eine teilweise durchbrochene Seitenansicht einer alternativen Ausführungsform eines chirurgischen Instruments;
- Figur 6:: eine perspektivische Ansicht eines weiteren Ausführungsbeispiels eines Endeffektors;
- Figur 7:: eine Längsschnittansicht des Endeffektors aus Figur 6;
- Figur 8:: eine Explosionsdarstellung des Endeffektors aus Figur 6 von oben gesehen;
- Figur 9:: eine Explosionsdarstellung des Endeffektors aus Figur 6 von unten gesehen;
- Figur 10:: eine perspektivische Ansicht eines weiteren, in Form eines Schneidwerkzeugs ausgebildeten Endeffektors;
- Figur 11:: eine Explosionsdarstellung des Endeffektors aus Figur 10 von oben gesehen;
- Figur 12:: eine Explosionsdarstellung des Endeffektors aus Figur 10 von unten gesehen;
- Figur 13:: eine perspektivische Ansicht eines weiteren, in Form eines Greifers ausgebildeten Endeffektors in einer Grundstellung;
- Figur 14:: eine Seitenansicht des in Figur 13 dargestellten Endeffektors;
- Figur 15:: eine Explosionsdarstellung des in Figur 13 dargestellten Endeffektors; und
- Figur 16:: eine Seitenansicht des in Figur 13 dargestellten Endeffektors in einer Zugstellung mit abgestützten Anlenkgliedern.

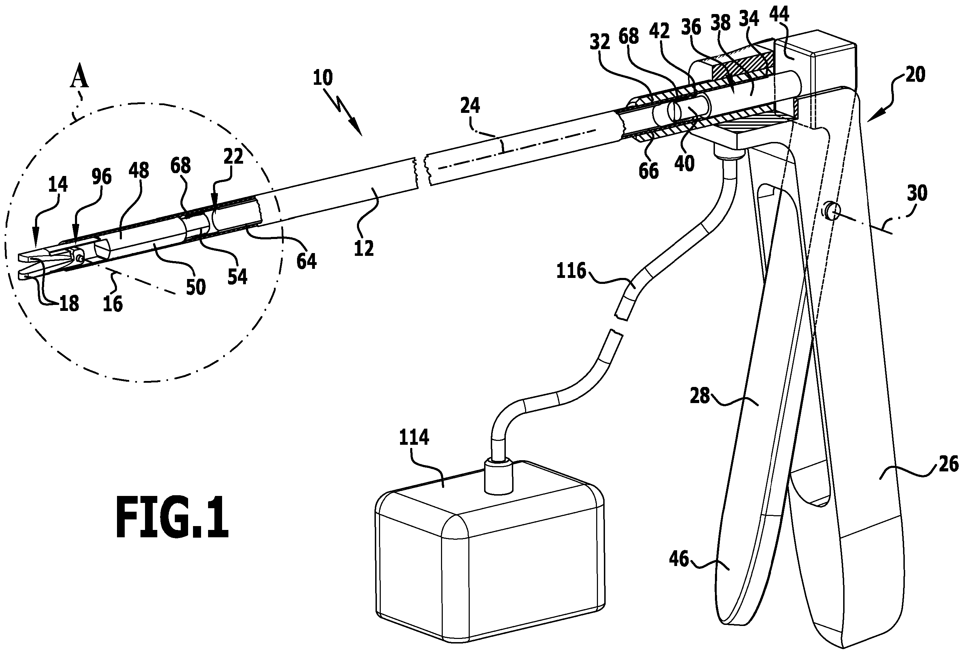

In Figur 1 ist ein insgesamt mit dem Bezugszeichen 10 versehenes chirurgisches Instrument dargestellt, umfassend einen langgestreckten rohrförmigen Schaft 12, einen an dessen distalem Ende angeordneten Endeffektor 14 mit zwei relativ zueinander um eine Schwenkachse 16 verschwenkbaren Werkzeugelementen 18 in Form von klemmbackenförmigen Maulteilen sowie eine am proximalen Ende des Instruments 10 angeordnete Betätigungseinrichtung 20 zum Einleiten und Übertragen einer Betätigungskraft auf ein insgesamt mit dem Bezugszeichen 22 versehenes Kraftübertragungsglied zum Übertragen der Betätigungskraft auf den Endeffektor 14 zum Bewegen der Werkzeugelemente 18.

Die Betätigungseinrichtung 20 umfasst einen im Wesentlichen quer zu einer vom Schaft 12 definierten Längsachse 24 abstehenden Griffteil 26, an welchem ein Griffelement 28 um eine von der Längsachse 24 beabstandete, jedoch senkrecht zu dieser verlaufende Schwenkachse verschwenkbar gelagert ist. Ein proximales Ende des Schafts 12 ist in einer in distaler Richtung vom Griffteil 26 abstehenden und koaxial zur Längsachse 24 angeordneten Führungshülse 32 gehalten. Der Schaft 12 kann fest mit der Führungshülse 32 oder lösbar mit dieser verbunden sein.

Im proximalen Endbereich 34 des Schafts 12 ist ein erster Kolben 36 parallel zur Längsachse 24 verschiebbar gelagert. Er umfasst einen ersten Kolbenabschnitt 38, dessen Außendurchmesser an einen Innendurchmesser des Schafts 12 angepasst ist. Vom ersten Kolbenabschnitt 38 steht in Richtung auf das distale Ende weisend ein zweiter Kolbenabschnitt 40 koaxial zur Längsachse 24 ab, dessen Außendurchmesser etwas kleiner ist als der Innendurchmesser des Schafts 12, so dass ein den zweiten Kolbenabschnitt 40 umgebender Ringraum 42 ausgebildet wird. Eine auf Höhe der Längsachse 24 ausgebildete und vom Griffelement 28 in distaler Richtung weisende Druckfläche 44 liegt an einer in proximaler Richtung weisenden Stirnfläche des Kolbens 36 an und kann durch Verschwenken des Griffelements 28 um die Schwenkachse 30 den Kolben 36 in distaler Richtung bewegen. So wird insbesondere auch eine gelenkige Verbindung der Betätigungseinrichtung 20 und des Kolbens 36 des Kraftübertragungsglieds 22 realisiert. Optional kann an der Betätigungseinrichtung 20 ein nicht dargestelltes Rückstellelement vorgesehen sein, welches das Griffelement 28 in eine Grundstellung überführt, wenn keine Druckkraft auf dieses ausgeübt wird, wobei in der Grundstellung die Druckfläche 44 vorzugsweise ihre proximalste Stellung einnimmt, was gleichzeitig bedeutet, dass ein am weitesten von der Längsachse 24 abstehender Betätigungsbereich 46 des Griffelements 28 maximal weit in distaler Richtung vorsteht und in diese weist.

Das Kraftübertragungsglied 22 ist mehrteilig ausgebildet und umfasst unter anderem den Kolben 36. Des Weiteren umfasst das Kraftübertragungsglied 22 einen weiteren Kolben 48, welcher ein distales Ende des Kraftübertragungsglieds 22 definiert. Der Kolben 48, welcher einen Kraftübertragungsabschnitt 49 bildet, der mit dem Kraftübertragungsglied 22 verbunden ist und ein distales Ende desselben bildet, ist im Wesentlichen identisch mit dem Kolben 36 aufgebaut und umfasst einen ersten zylindrischen Kolbenabschnitt 50, welcher einen Außendurchmesser aufweist, welcher einem Innendurchmesser des Schafts 12 entspricht, so dass der Kolben 48 im Wesentlichen spielfrei im Schaft parallel zur Längsachse 24 bewegbar gelagert ist. Von einer in proximaler Richtung weisenden Stirnfläche 52 des ersten Kolbenabschnitts 50 steht ein zweiter Kolbenabschnitt 54 koaxial zur Längsachse 24 in proximaler Richtung weisend ab. Die Länge des zweiten Kolbenabschnitts 54 beträgt etwa ein Drittel der Länge des ersten Kolbenabschnitts 50. Den zweiten Kolbenabschnitt 54 umgebend ist ein Ringraum 56 ausgebildet. Eine in proximaler Richtung weisende Stirnfläche 58 des zweiten Kolbenabschnitts 54 definiert eine Druckfläche. Kanten des zylindrischen zweiten Kolbenabschnitts 54 sind abgerundet.

Zwischen den beiden Kolben 36 und 48 erstreckt sich ein insgesamt mit dem Bezugszeichen 60 versehener Fluidabschnitt. Er umfasst einen eine Innenwand 62 des Schafts im Wesentlichen zwischen den zweiten Kolbenabschnitten 40 und 54 auskleidenden Schlauch 64, dessen freie proximale und distale Enden 66 jeweils mit einem identisch ausgebildeten Dichtungselement 68 verschlossen sind. Nachfolgend beschränkt sich daher die Beschreibung der Ankopplung der Kolben 36 und 48 an den Fluidabschnitt 60 auf die Ankopplung des Kolbens 48 an den Fluidabschnitt 60, die eine sogenannte Kolben-Dichtungselement-Anordnung definieren.

Der Schlauch 64 ist vorzugsweise fest mit dem Schaft 12 verbunden und relativ zu diesem um die Längsachse 24 unverdrehbar. Er kann selbst einen Schaft bilden, wenn auf den Schaft 12 verzichtet wird. Das Dichtungselement 68 ist direkt an den Schlauch 64 angeformt oder mit diesem fest verbunden und schließt sich direkt an das jeweilige Ende 66 an. Das Dichtungselement 68 umfasst einen ersten, im Wesentlichen hülsenförmigen Abschnitt 70, welcher knapp doppelt so lang ist wie eine Länge des zweiten Kolbenabschnitts 54 parallel zur Längsachse 24. Ein freies, vom Ende 66 weg weisendes Ende des Abschnitts 70 ist durch einen zweiten Abschnitt 74 des Dichtungselements 68 verschlossen. Der Abschnitt 74 erstreckt sich im Wesentlichen quer zur Längsachse 24 und definiert einen Flächenbereich, welcher im Wesentlichen der Größe einer inneren freien Querschnittsfläche des Schafts 12 entspricht. Das Dichtungselement 68 ist flexibel, jedoch inelastisch und insgesamt in Form einer fluiddichten Membran ausgebildet, die einen sogenannten Rollbalg bildet. Es weist einen vom jeweiligen Kolben weg weisenden ringförmigen Rand 76 auf, der im vorliegenden Fall fest mit dem Ende 66 des Schlauchs 64 und damit indirekt fest mit der Innenwand 62 verbunden ist.

Der Schlauch 64 mit den beidseitig ausgebildeten Dichtungselementen 68 definiert einen Fluidraum 78, welcher mit einem Fluid 80, vorzugsweise einer inkompressiblen Flüssigkeit, gefüllt ist. Der Fluidraum 78 wird vorzugsweise gasfrei befüllt, das bedeutet, dass insbesondere keine Luftblasen oder Gasblasen im Fluidraum 78 enthalten sind. In jedem Fall ist das Fluid 80 dauerhaft in den Fluidraum 78, welcher vorzugsweise leckagefrei ist, eingefüllt. Ein Volumen des Fluids 80 entspricht in etwa einem vom Schlauch 64 definierten Volumen, das heißt dem Volumen, das definiert wird durch das Produkt der inneren Querschnittsfläche des Schlauchs 64 und dessen Länge.

Die zweiten Abschnitte 74 der Dichtungselemente 68 können optional mit den zweiten Kolbenabschnitten 40 und 54 verbunden sein, beispielsweise mit der Stirnfläche 58 des zweiten Kolbenabschnitts 54. Das Dichtungselement 68 weist eine Wandstärke auf, die kleiner als 0,5 mm ist, vorzugsweise kleiner als 0,1 mm, und je nach Materialwahl auch weniger als 0,05 mm betragen kann. Das Verhältnis zwischen einer Länge des ersten Abschnitts 70 und einem Durchmesser des zweiten Abschnitts 74 beträgt bei dem in den Figuren dargestellten Ausführungsbeispiel etwa 2. Es kann jedoch kleiner als 1, insbesondere auch kleiner als 0,5 oder 0,2 sein.

Das Dichtungselement 68 ist derart ausgebildet, dass der zweite Abschnitt 74 umstülpbar ist unter Ausbildung eines vom Rand 76 weg weisenden und in einer vom Rand 76 weg weisenden Richtung geschlossenen doppellagigen gerollten Dichtungsabschnitts 82. Dies bedeutet, dass der erste Abschnitt 70 in Richtung auf der Längsachse 24 auf sich selbst zurück gefaltet oder umgeschlagen ist, so dass ein erster Teil des ersten Abschnitts 70 am Schaft 12 anliegt, ein zweiter Teil des ersten Abschnitts 70 an einer Außenfläche des zweiten Kolbenabschnitts 54. Ingesamt taucht der Dichtungsabschnitt 82 in den Ringraum 56 ein, ohne darin zu verklemmen. Dies ermöglicht es, dass der zweite Abschnitt 74 parallel zu sich selbst in Richtung der Längsachse 24 bewegbar ist, sowohl in distaler als auch proximaler Richtung. Bei einer Bewegung des zweiten Abschnitts 74 beispielsweise in distaler Richtung rollt sich der teilweise umgeschlagene erste Abschnitt 70 ab und kann in einer distalsten Stellung des Kolbens 48 vollständig abgerollt sein und die Innenwand 62 in Form eines zylindrischen Abschnitts des Dichtungselements 68 auskleiden. Umgekehrt kann der zweite Abschnitt 74 bis an den Rand 76 bewegt werden, wobei der erste Abschnitt 70 dann so umgestülpt ist, dass der Dichtungsabschnitt 82 in etwa die Hälfte der Länge des ersten Abschnitts 70 aufweist. Grundsätzlich ist es denkbar, die Abmessungen der Teile relativ zueinander so zu wählen, dass der zweite Abschnitt 74 in proximaler Richtung über den Rand 76 in Richtung auf die Betätigungseinrichtung 20 hin verschoben werden kann. Dies ist prinzipiell möglich, bis der erste Abschnitt 70 wieder ganz abgerollt ist. Damit ergibt sich theoretisch ein maximaler Verschiebeweg des Kolbens 48, welcher einer doppelten Länge des ersten Abschnitts 70 entspricht.

Die beschriebene Anordnung aus Kolben 48 und Dichtungselement 68 ist analog auch am Kolben 36 vorgesehen. Dies ist in Figur 1 schematisch gestrichelt eingezeichnet. Die Kolben 36 und 48 werden vorzugsweise so angeordnet, dass in einer Grundstellung des Fluidabschnitts 60 die zweiten Kolbenabschnitte 40 und 54 an den zweiten Abschnitten 74 anliegen, die beide jeweils in etwa auf Höhe der Ränder 76 angeordnet sind. Der Fluidraum 78 ist dann, wie beschrieben, blasenfrei mit dem Fluid 80 befüllt, welches insbesondere auch die ringraumartigen Abschnitte im Bereich der Dichtungsabschnitte 82 ausfüllt.

Wird der Betätigungsbereich 46 vom Griffelement 28 in proximaler Richtung verschwenkt, dann wird die Druckfläche 44 in distaler Richtung bewegt und schiebt den Kolben 36 ebenfalls in distaler Richtung vor. Gleichzeitig wird der am zweiten Kolbenabschnitt 40 anliegende zweite Abschnitt 74 des Dichtungselements 68 in distaler Richtung bewegt, und zwar distalseitig des Rand 76. Aufgrund der Inkompressibilität des Fluids 80 wird infolge der Bewegung des Kolbens 36 der Kolben 48 in distaler Richtung bewegt. Dabei rollt sich der Dichtungsabschnitt 82 ab, der an der Innenwand 62 des Schafts 12 anliegende und sich daran abstützende Teil des Dichtungsabschnitts 82 wird immer länger und der am zweiten Kolbenabschnitt 54 anliegende und sich daran abstützende Teil des Dichtungsabschnitts 82 wird immer kürzer, bis der erste Abschnitt 70 vollständig abgerollt ist und der zweite Abschnitt 74 ein distales Ende des Dichtungselements 68 bildet. In umgekehrter Weise führt eine Bewegung des Kolbens 48 in proximaler Richtung zu einer Bewegung des Kolbens 36 ebenfalls in proximaler Richtung. Sind die Abschnitte 74 mit den jeweiligen zweiten Kolbenabschnitten 40 und 54 verbunden, kann durch eine Bewegung des Kolbens 36 in proximaler Richtung der Kolben 48 ebenfalls in proximale Richtung gezogen werden.

Der Endeffektor 14 ist beim Instrument 10 direkt am Kolben 48 angeformt. Grundsätzlich wäre es möglich, den Kolben 48 und den Endeffektor 14 mit den Werkzeugelementen 18 einstückig auszubilden. Leichter herzustellen wird der Endeffektor 14 in Verbindung mit dem Kolben 48 jedoch, wenn zwei identische Teile ausgebildet werden, die mittels einer durch einen die Schwenkachse 16 und eine Lagerwelle 84 definierenden Stift am Schaft gehalten sind, welcher beispielsweise in koaxial zur Schwenkachse 16 orientierten Bohrungen in einer Wand des Schafts 12 gelagert ist. Auf der Lagerwelle 84 gelagert sind proximale Endabschnitte 86 der Werkzeugelemente, die im Wesentlichen würfelförmige Lagerbacken 87 bilden und mit einer korrespondierend zur Lagerwelle 84 ausgebildeten und angeordneten Bohrung 88 versehen sind. Die beiden Endabschnitte 86 der Werkzeugelemente 18 sind nebeneinander auf der Lagerwelle 84 angeordnet. Voneinander weg weisende Außenflächen 89 der Lagerbacken 87 sind konvex gekrümmt und definieren vorzugsweise Kugeloberflächen mit einem Radius, welcher einem Radius des Schafts 12 entspricht.

Mit dem jeweiligen Endabschnitt 86 starr verbunden ist ein Werkzeugende 90 des jeweiligen Werkzeugelements 18. Die Lagerbacken 87 sind etwa nur halb so breit wie ein proximales Ende des Werkzeugendes 90, so dass, wenn sich die Werkzeugenden 90 überdecken, die Lagerbacken 87 nebeneinander auf der Lagerwelle 84 verschwenkbar gelagert sind.

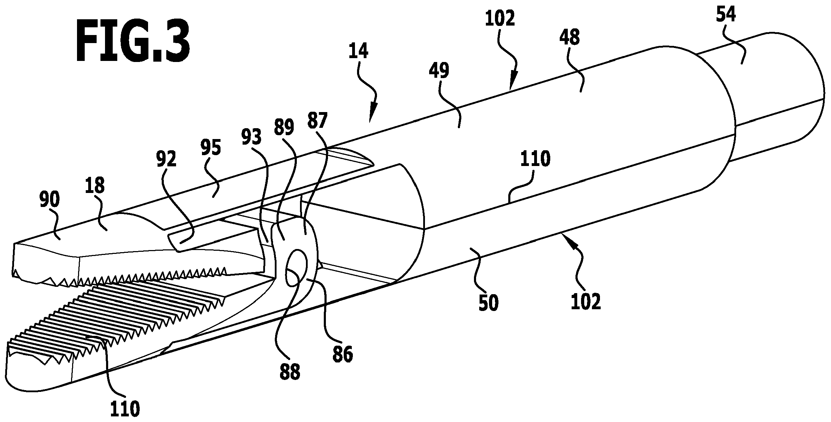

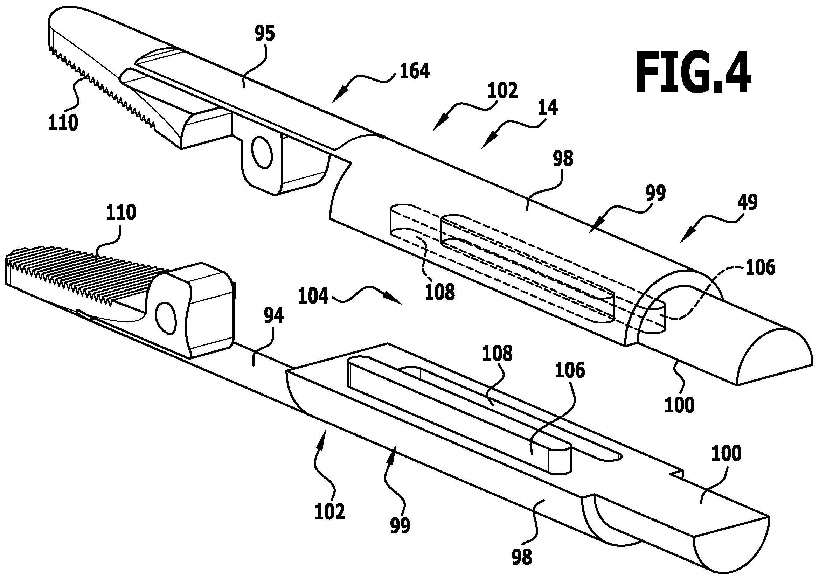

Beim vorliegenden Instrument 10 sind Werkzeugselemente 90 in Form von Klemmbacken 91 ausgebildet, die etwa so breit sind, wie ein Durchmesser des Schafts 12 und deren aufeinander zu weisende Innenflächen, wie in den Figuren 3 und 4 dargestellt, optional mit einer Querverzahnung 110 versehen sein können, welche eine Verzahnungshöhe in einem Bereich von 50 µm bis 400 um, vorzugsweise 200 µm bis 300 µm, aufweisen kann. Etwa in der Mitte zwischen distalem Ende des Werkzeugendes 90 und der Schwenkachse 16 ist durch einen Spalt 92 ein eine Außenfläche des Werkzeugelements 18 definierender, flacher lang gestreckter Steg 94 ausgebildet, dessen proximales Ende einstückig mit dem Kolben 48 ausgebildet ist. Der Steg 94 ist flexibel und elastisch biegbar und bildet ein Anlenkglied 95 in Form eines Festkörpergelenks 96 aus. Der Steg 94 erstreckt sich in etwa gleich weit in distaler wie in proximaler Richtung bezogen auf die Schwenkachse 16. So wird ferner eine Durchbrechung definiert, die distalseitig begrenzt wird durch die Lagerbacken 87, proximalseitig vom ersten Kolbenabschnitt 50 und in radialer Richtung von den einander gegenüberliegenden Anlenkgliedern 95. Ein Abstand zwischen einem proximalen Ende der Lagerbacken 87 und einem distalen Ende des ersten Kolbenabschnitts 50 ist etwa zwei- bis dreimal so groß wie eine Erstreckung der Lagerbacken 87 in einer Richtung parallel zur Längsachse 24. Um eine optimale Bewegbarkeit der Werkzeugelemente 18 zu erreichen und ein Verklemmen derselben auf der Lagerwelle 84 zu verhindern, sind proximale Enden der Werkzeugenden 90 durch einen schmalen Spalt von der Lagerbacke 87 des jeweils anderen Werkzeugelements 18 getrennt.

Die Herstellung sowohl des Kolbens 48 als auch den Endeffektors 14 wird vereinfacht, wenn zwei identische Endeffektorteile 102 ausgebildet werden, die ineinander überführt werden können durch Rotation um die Längsachse 24. Hierzu wird der Kolben 48 aus zwei Kolbenhälften 98, die jeweils eine Abschnittshälfte 99 des Kraftübertragungsabschnitts 49 bilden, zusammengesetzt, die die Längsachse 24 enthaltende Verbindungsflächen 100 definieren, die parallel zu den Stegen 94 verlaufen und den Kolben 48 mit seinen ersten und zweiten Kolbenabschnitten 50 und 54 längs halbieren. Zum Verbinden der beiden Endeffektorteile 102 dient eine insgesamt mit dem Bezugszeichen 104 versehene Verbindungseinrichtung, welche jeweils zwei Verbindungselemente 106 und 108 tragen. Das Verbindungselement 106 ist in Form eines langgestreckten, sich parallel zur Längsachse 24 erstreckenden Vorsprungs ausgebildet. Das andere Verbindungselement 108 ist in Form einer zum Vorsprung korrespondierenden Ausnehmung ausgebildet, in welche das Verbindungselement 106 formschlüssig eingreifen kann. Die Verbindungselemente 106 und 108 sind auf den Verbindungsflächen 100 jeweils so angeordnet, dass die beiden Abschnittshälften 99 in einer Verbindungsstellung den Kraftübertragungsabschnitt 49 bilden. Die Kolbenhälften 98 sind optional fest miteinander verbunden, beispielsweise durch Kleben oder Schweißen.

Das Anlenkglied 95 weist eine Materialstärke auf, die abhängig vom Durchmesser des Kraftübertragungsabschnitts 49 in einem Bereich von etwa 50 µm bis 700 µm liegt. Bei einem Innendurchmesser des Schafts 12 von etwa 2 mm beträgt die Dicke des Stegs 94 etwa 100 µm.

Die Funktionsweise des Endeffektors 14 wird nachfolgend näher erläutert.

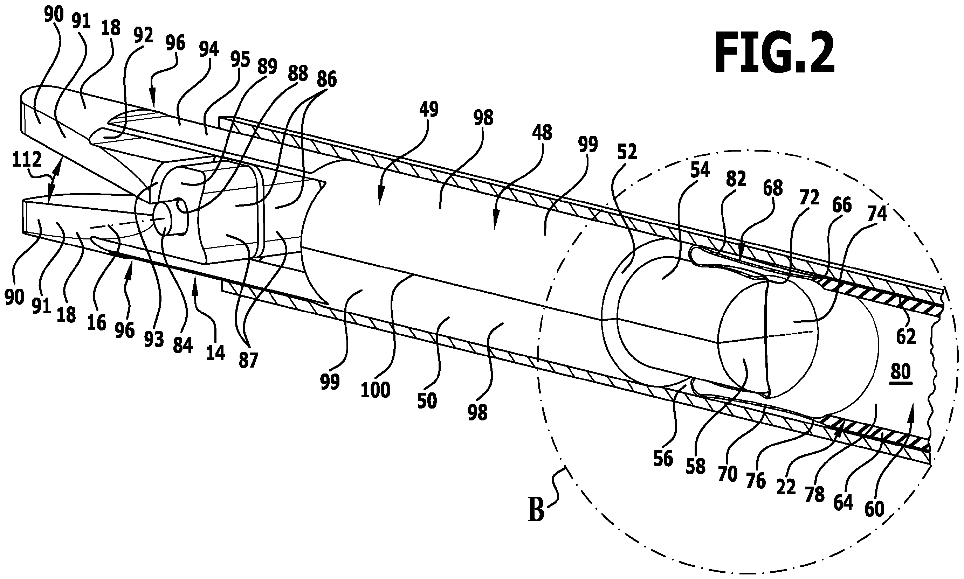

In den Figuren 1 und 2 ist der Endeffektor 14 in einer Grundstellung dargestellt. Die Werkzeugenden 90 sind etwas aufgespreizt und definieren zwischen sich einen Öffnungswinkel 112 von ungefähr 30°. Die Stege 94 verlaufen in der Grundstellung parallel zur Längsachse 24. Wird der Kolben 48 in proximaler Richtung bewegt, werden die Stege 94 ebenfalls in proximaler Richtung gezogen. Da die Werkzeugelemente 90 starr verschwenkbar um die Lagerwelle 84 gelagert sind, verformt sich der Steg 94 und damit das Festkörpergelenk 96, so dass die Werkzeugenden 90 von der Längsachse 24 weg nach außen geschwenkt werden. Damit kann der Öffnungswinkel 112 vergrößert werden, was das Fassen von Gewebe mit dem Endeffektor 14 vereinfacht. Durch entsprechende Fertigung ist es auch möglich, in einer Grundstellung den Steg 94 etwas zu verformen, so dass ohne über den Kolben 48 eingeleitete Kräfte die jeweiligen Werkzeugelemente 90 weiter auseinander verschwenkt sind.

Wird der Kolben 48 in distaler Richtung bewegt, wird ebenfalls der Steg 94 in distaler Richtung verschoben. Da die Werkzeugenden 90 keine andere Bewegungsmöglichkeit haben als aufeinander zu verschwenkt zu werden, geschieht dies unter entsprechender Verformung, das heißt elastischer Verbiegung des Stegs 94 und damit des Festkörpergelenks 96. Ein Verschiebeweg des Kolbens 48 wird begrenzt durch die Werkzeugenden 90, deren Innenflächen in einer Schließstellung, die in den Figuren nicht dargestellt ist, aneinander anliegen oder im Wesentlichen aneinander anliegen.

Die Festkörpergelenke 96 ermöglichen eine nahezu spielfreie Lagerung und Kraftübertragung vom Kraftübertragungsglied 22 auf die Werkzeugelemente 18 zum Bewegen derselben, das heißt im vorliegenden Fall zum Verschwenken um die Schwenkachse 16.

Zum Bewegen des Kolbens 48 in distaler und proximaler Richtung kann, wie oben beschrieben, der Kolben 36 vorgesehen werden. Optional kann man auf den Kolben 36 mit zugehörigem Dichtungselement 68 auch verzichten, wenn eine alternative Fluidzuführeinrichtung 114, zum Beispiel in Form einer externen Fluidpumpe, welche über eine Zuleitung 116 mit einem proximalen Ende des Schafts 12 des darin angeordneten Schlauchs 64 fluiddicht verbunden ist, vorgesehen ist. Mit der Fluidzuführeinrichtung 114 kann das Fluid 80 in das proximale Ende des Schafts 12 gedrückt und damit der Kolben 48 in distaler Richtung bewegt werden. Ein Entfernen oder eine Reduzierung des Drucks im Fluid 80 führt dann aufgrund der elastisch federnd ausgebildeten Festkörpergelenke 96 zu einer Rückverschwenkung der Werkzeugelemente 18 in ihre Grundstellung und damit verbunden durch die Verbindung des Kolbens 48 über die Stege 94 mit den Werkzeugelementen 18, zu einer Rückbewegung des Kolbens 48 in proximaler Richtung.

Eine Betätigung der Fluidzuführeinrichtung 114 kann beispielsweise über das verschwenkbar gelagerte Griffelement 28 realisiert werden, wobei vorzugsweise ein elektrisches, elektronisches oder elektromechanisches Schaltungselement zum Betätigen der Fluidzuführeinrichtung 114 infolge einer Verschwenkbewegung des Griffelements 28 vorgesehen ist, beispielsweise in Form eines nicht dargestellten Schalters, Tasters oder elektronischen Schaltelements wie zum Beispiel eines optischen Sensors oder eines induktiven oder kapazitiven Näherungssensors, welcher an der Betätigungseinrichtung 20 angeordnet ist zum Detektieren einer Bewegung des Griffelements 28 relativ zum Griffteil 26.

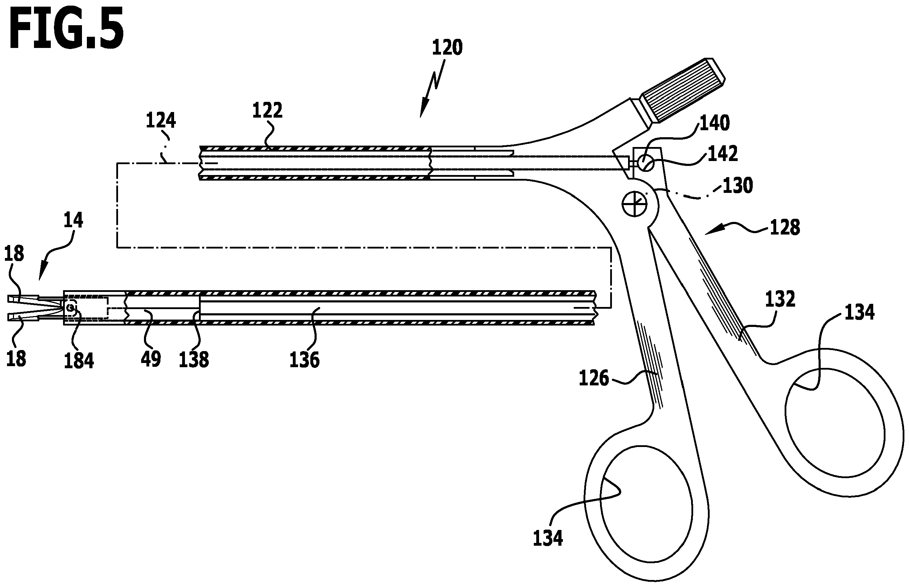

In Figur 5 ist eine alternative Ausführungsform eines chirurgischen Mikrorohrschaftinstruments dargestellt. Es umfasst einen langgestreckten rohrförmigen Schaft 122, an dessen distalem Ende der vom Instrument 10 bekannte und oben beschriebene Endeffektor 14 montiert ist, so dass zu Einzelheiten betreffend den Aufbau des Endeffektors 14 auf die obige Beschreibung verwiesen werden kann.

Ein proximales Ende des Schafts 122 ist in etwa rechtwinklig bezogen auf eine Längsachse 124 des Schafts 122 abgewinkelt und bildet ein erstes Griffteil 126 einer Betätigungseinrichtung 128. Um eine quer zur Längsachse 124 orientierte, jedoch von dieser beabstandete Schwenkachse 130 ist relativ zum Griffteil 126 ein zweites Griffteil 132 verschwenkbar gelagert. Freie Enden der Griffteile 126 und 132 sind jeweils mit einem Fingerring 134 versehen, durch den eine Bedienperson einen oder mehrere Finger durchstecken kann.

Im Schaft parallel zur Längsachse 124 verschiebbar gelagert, ist ein Kraftübertragungsglied in Form einer Schub- und Zugstange 136, deren distales Ende 138 fest mit dem Kraftübertragungsabschnitt 49 des Endeffektors 14 verbunden ist. Ein proximales Ende der Schub- und Zugstange 136 ist in Form eines Kugelkopfs 140 ausgebildet, welcher in einer entsprechenden Aufnahme 142 eines über die Schwenkachse 130 hinausragenden Abschnitts des Griffteils 132 gelagert ist zur Ausbildung einer kugelgelenkigen Verbindung des Schubund Zugglieds 136 mit dem Griffteil 132. Durch Verschwenken des Griffteils 132 relativ zum Griffteil 126 lässt sich die Schub- und Zugstange 136 in distaler Richtung bewegen zum Schließen der Werkzeugelemente 18 sowie in proximaler Richtung zum Öffnen der Werkzeugelemente 18.

Das in Figur 5 dargestellte Instrument 120 dient als Beispiel dafür, dass es möglich ist, den im Zusammenhang mit dem Instrument 10 beschriebenen Endeffektor 14, ebenso wie auch alle anderen nachfolgend noch beschriebenen Endeffektoren, mit sogenannten herkömmlichen Rohrschaftinstrumenten zu kombinieren, das heißt mit Instrumenten, die als Kraftübertragungsglied eine stabförmige Schub- und Zugstange 136 oder dergleichen aufweisen. Die Lagerwelle 84 ist in entsprechenden Sacklöchern oder Bohrungen des Schafts 122 gehalten.

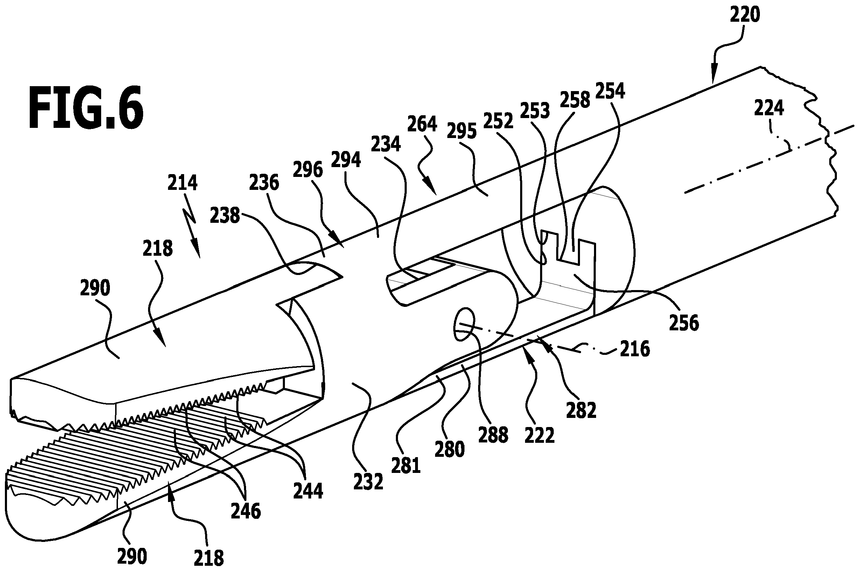

Ein weiteres Ausführungsbeispiel eines Endeffektors, welcher sowohl am Instrument 10 als auch am Instrument 120 vorgesehen werden kann, ist beispielhaft in den Figuren 6 bis 9 dargestellt und insgesamt mit dem Bezugszeichen 214 versehen.

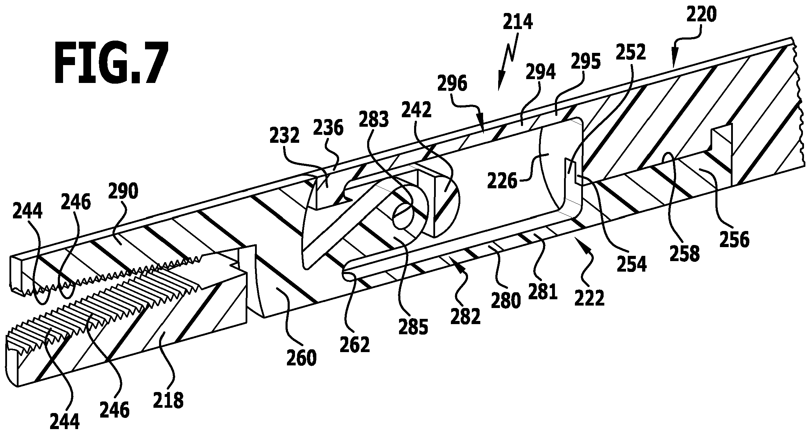

Der Endeffektor 214 umfasst zwei um eine von einer nicht dargestellten und an einem Schaft gehaltenen Lagerwelle definierte Schwenkachse 216 gegeneinander verschwenkbar gelagerte Werkzeugelemente 218. Insgesamt ist der Endeffektor 214 zweiteilig ausgebildet und umfasst einen ersten Endeffektorteil 220 und einen zweiten Endeffektorteil 222. Die beiden Endeffektorteile 220 und 222 sind, anders als die Endeffektorteile 102, nicht identisch ausgebildet. Vielmehr ist jeder Endeffektorteil für sich betrachtet, ebenfalls anders als die Endeffektorteile 102, symmetrisch zu einer Spiegelebene ausgebildet, welche senkrecht zur Schwenkachse 216 verläuft und die Längsachse 224 des Endeffektors 214 enthält. Eine Schnittansicht des Endeffektors 214 längs dieser Spiegelebene ist in Figur 7 dargestellt.

Der Endeffektorteil 220 umfasst einen zylindrischen Kraftübertragungsabschnitt 249, welcher auch als Kolben bezeichnet werden kann. Ein proximales Ende desselben ist analog dem Kolben 48 geformt zur Verwendung in einem Instrument 10 oder ist mit einer Schub- und Zugstange 136 wie beim Instrument 120 verbunden. Der Kraftübertragungsabschnitt 249 definiert eine in distaler Richtung weisende Stirnfläche 226. Vom Kraftübertragungsabschnitt 249 steht in distaler Richtung ein Steg 294 ab, der ein flexibles Anlenkglied 295 zur Ausbildung eines Festkörpergelenks 296 definiert. Eine von der Längsachse 224 weg weisende Außenfläche 228 des Stegs 294 bildet eine Fortsetzung einer zylindrischen Außenfläche des Kraftübertragungsabschnitts 249. Eine in Richtung auf die Längsachse 224 hin weisende Innenfläche 230 des Stegs 294 ist eben.

Das Anlenkglied 295 ist an einen zylindrischen Zugabschnitt 232 des Werkzeugelements 218 angeformt. Ein sich parallel zum Steg 294 erstreckender Spalt trennt diesen von zwei Lagerbacken, die wiederum durch einen Spalt 234 voneinander beabstandet sind. Die Lagerbacken 287 weisen in proximale Richtung und sind koaxial zur Schwenkachse 216 mit einer Bohrung 283 versehen, durch die eine stiftförmige Lagerwelle durchgesteckt werden kann, um das Werkzeugelement 218 des Endeffektorteils 220 verschwenkbar an einem Schaft zu lagern. Etwas distalseitig eines Verbindungsbereichs zwischen dem Steg 294 und dem Zugabschnitt 232 ist der Zugabschnitt 232 mit einer Durchbrechung 238 versehen, welche symmetrisch zur Symmetrieebene und quer zur Längsachse 224 den Zugabschnitt 232 in etwa quaderförmig durchbricht und eine in distale Richtung weisende Stirnfläche 240 des Zugabschnitts 232 eröffnet. Der Spalt 234 und die Durchbrechung 238 sind miteinander verbunden. Der Spalt 234 trennt die beiden Lagerbacken 287 nicht vollständig, sondern endet kurz vor einem proximalen Ende derselben, so dass ein in proximaler Richtung weisender, die Lagerbacken 287 verbindender Verbindungssteg 242 ausgebildet wird. Vom Zugabschnitt 232 steht in distaler Richtung weisend ein Werkzeugende 290 ab, und zwar derart, dass eine virtuelle Verlängerung desselben in proximaler Richtung dem Steg 294 diametral gegenüberliegen würde. Eine in Richtung auf die Längsachse 224 hin weisende Klemmfläche 244 ist mit einer Querverzahnung 246 versehen, welche eine Höhe in einem Bereich zwischen 20 µm und 100 µm aufweisen kann.

Zum Verbinden des Endeffektorteils 220 mit dem Endeffektorteil 222 dient eine Verbindungseinrichtung 248 mit einem ersten Verbindungselement 252, welches am Kraftübertragungsabschnitt 249 ausgebildet ist, und zwar in Form einer quaderförmigen Ausnehmung, die in einer von der Längsachse 224 weg weisenden radialen Richtung geöffnet ist. Von einem Boden 253 der Ausnehmung steht eine langgestreckte quaderförmige Leiste 254 ab, die in einer Richtung parallel zur Längsachse 224 etwas kürzer ist als die Ausnehmung, jedoch bündig mit der Stirnfläche 226 abschließt. Eine Breite quer zur Längsachse 224 des Verbindungselements 252 entspricht einer Breite der Durchbrechung 238 sowie des Spalts 234.

Die Verbindungseinrichtung 248 umfasst ein zweites Verbindungselement 256, welches derart geformt ist, dass es die vom Verbindungselement 252 definierte Ausnehmung vollständig ausfüllt. Es ist daher insgesamt etwa quaderförmig und weist einen äußeren Oberflächenabschnitt auf, welcher den Kraftübertragungsabschnitt 249 zum Zylinder ergänzt. Zur Aufnahme der Leiste 254 ist eine Nut 258 am Verbindungselement 256 ausgebildet, welche proximalseitig verschlossen ist. So kann die Leiste 254 in die Nut 258 eingreifen, eine Relativbewegung der beiden Verbindungselemente 252 und 256 relativ zueinander parallel zur Längsachse 224 wird jedoch verhindert.

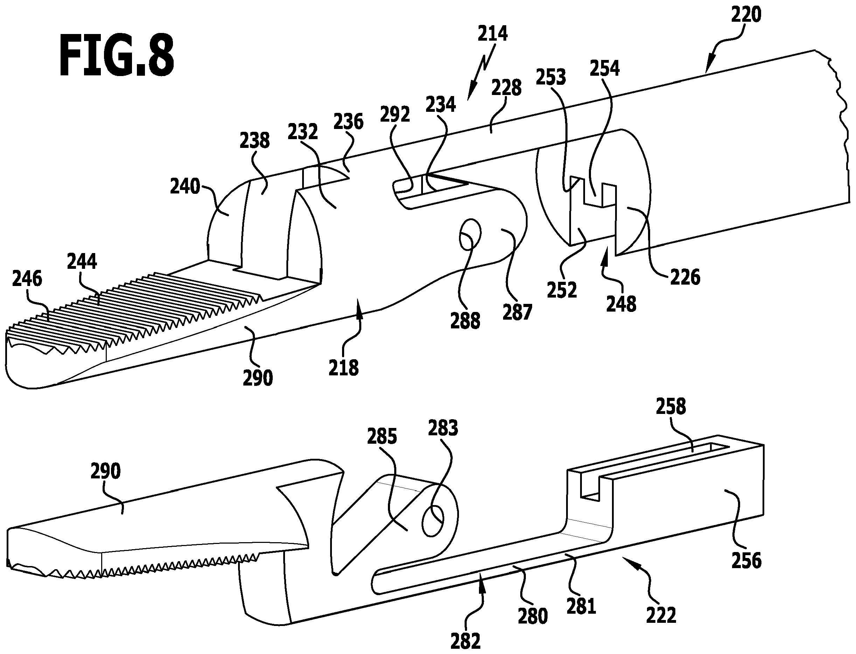

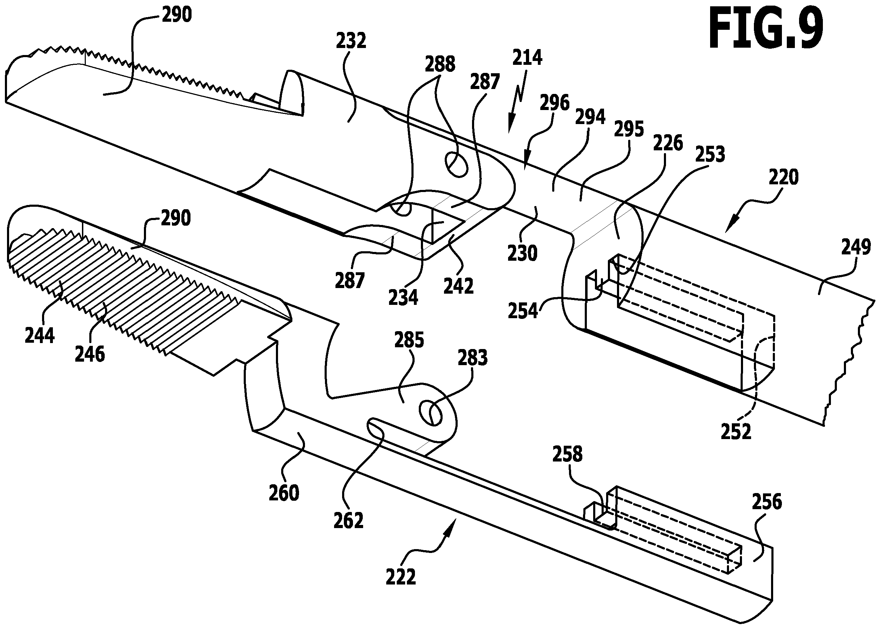

Dem Steg 294 diametral gegenüberliegend erstreckt sich vom Verbindungselement 256 ein Steg 280 in distaler Richtung, welcher ein Anlenkglied 281 definiert, welches ein Festkörpergelenk 282 zum Übertragen von Schub- und Zugkräften vom Kraftübertragungsabschnitt 249 auf das vom zweiten Endeffektorteil 222 umfasste Werkzeugelement 218 ermöglicht. Der Steg 280 endet an einem backenförmigen Zugabschnitt 260, von dem in proximaler Richtung und schräg auf die Längsachse 224 hin weisend ein Lagerbacken 285 absteht, welcher mit einer Bohrung 283 versehen ist. Der Lagerbacken 285 ist durch einen kurzen, sich in distaler Richtung etwas über die Schwenkachse 216 hinaus erstreckenden Spalt 262 vom Steg 280 getrennt. Der Zugabschnitt 260, der Steg 280 und der Lagerbacken 285 sind alle gleich breit, wobei ihre Breite einer Breite des Verbindungselements 256 und damit einer Breite der Durchbrechung 238 entspricht. Dies ermöglicht es, zu einer Montage des Endeffektors 214 die getrennt voneinander gefertigten Endeffektorteile 220 und 222 derart zusammen zu bauen, dass die, wie in den Figuren 8 und 9 dargestellten, voneinander getrennten Endeffektorteile 220 und 222 durch Einführen des Verbindungselements 256 in die Durchbrechung 238 von oben her kommend, das heißt in Richtung auf die Klemmfläche 244 und die Längsachse 224 hin weisend, das Verbindungselement 256 durch die Durchbrechung 238 durchgeschoben werden kann, bis es auf der anderen Seite hervorsteht. Dann werden die Endeffektorteile 220 und 222 relativ zueinander derart verschwenkt, dass das Verbindungselement 256 in das Verbindungselement 252 eingreifen kann. Die Bohrungen 283 und 288 sind dann fluchtend ausgerichtet und ein sich vom Zugabschnitt 260 in distaler Richtung erstreckendes Werkzeugende 290, welches dann eine Grundstellung einnimmt, wie sie in den Figuren 6 und 7 dargestellt ist, ist symmetrisch und dem anderen Werkzeugende 290 gegenüberliegend angeordnet. Der Endeffektor 214 kann dann mit einer nicht dargestellten Lagerwelle an einem Schaft des Instruments festgelegt werden. Optional können die Endeffektorteile 220 und 222 im Bereich der Verbindungseinrichtung 248 verklebt und/oder verschweißt werden. Wird der Kraftübertragungsabschnitt 249 in einem hülsenförmigen Schaft geführt, verhindert dieser eine Trennung der beiden Teile, so dass eine Verklebung oder Verschweißung nicht zwingend erforderlich ist.

Der Endeffektor 214 ist aufgrund der durchdringenden oder durchgreifenden Anordnung der Werkzeugelemente 218 derart ausgebildet, dass eine von einem Kraftübertragungsglied eingeleitete Zugkraft zu einer Schließbewegung des Endeffektors führt, das heißt die Klemmflächen 244 werden gegeneinander geschwenkt. Eine Bewegung des Kraftübertragungsabschnitts 249 in distaler Richtung führt zu einer Verschwenkung der Werkzeugelemente 218 von der Längsachse 224 weg, so dass sich ein Öffnungswinkel zwischen den Klemmflächen 244 der Werkzeugelemente 218 wieder vergrößern lässt. Die beschriebene Schließbewegung auf Zug der Werkzeugelemente 218 wird insbesondere dadurch ermöglicht, weil die Angriffspunkte der Anlenkglieder 281 und 295 jeweils bezogen auf die Schwenkachse 216 auf anderen Seiten liegen als die Verbindungsbereiche der Werkzeugenden 290 an den Zugabschnitten 232 beziehungsweise 260.

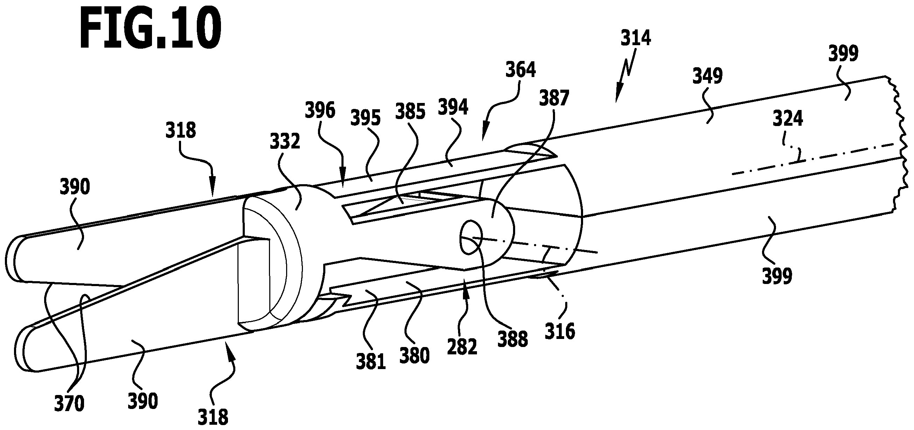

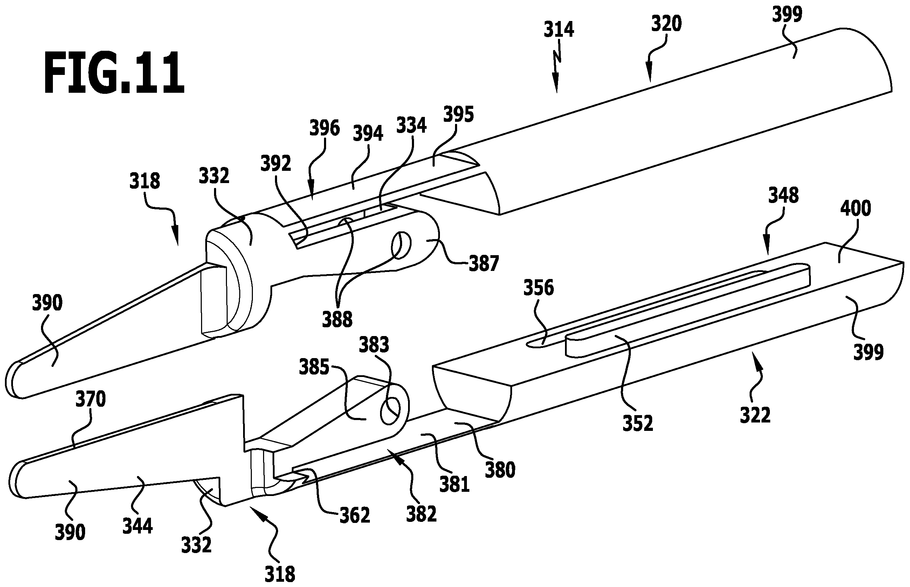

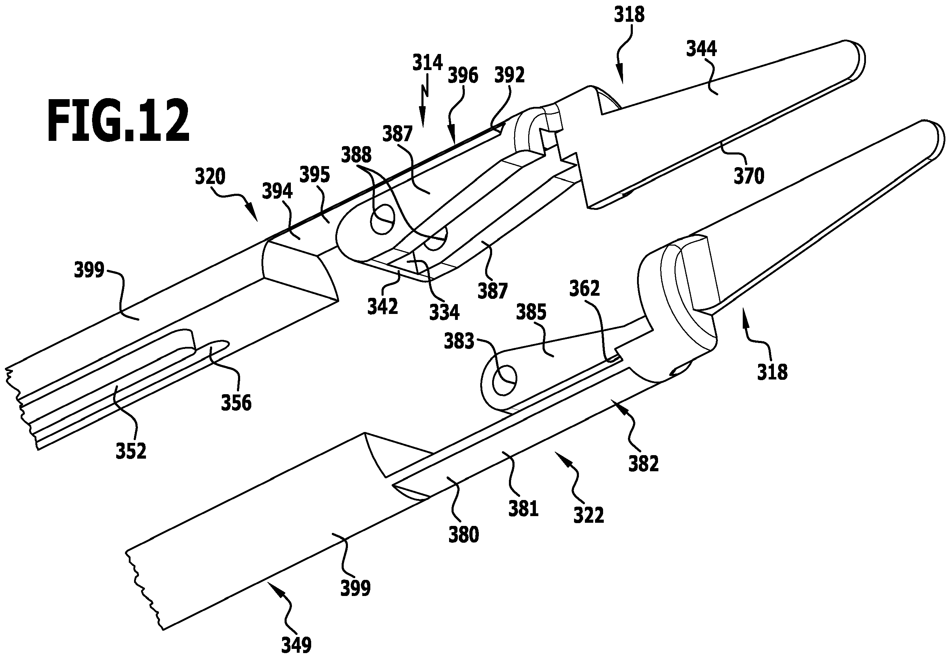

Ein weiteres Ausführungsbeispiel eines in Form einer Schneideinrichtung ausgebildeten Endeffektors ist in den Figuren 10 bis 12 beispielhaft dargestellt und insgesamt mit dem Bezugszeichen 314 versehen. Der Aufbau des Endeffektors 314 wird im Wesentlichen realisiert durch eine Kombination der Ausgestaltungen der Endeffektoren 14 und 214. Auch der Endeffektor 314 ist zweiteilig aufgebaut und umfasst zwei nicht identisch ausgebildete Endeffektorteile 320 und 322. Distale Enden derselben werden gebildet durch Werkzeugenden 390 in Form von aneinander abgleitenden Schneiden, die eine Materialstärke in einem Bereich von 200 µm bis 800 µm aufweisen können.

Der Kraftübertragungsabschnitt 349 ist insgesamt zylindrisch ausgebildet und umfasst zwei identisch ausgebildete Abschnittshälften 399. Diese sind, wie die Abschnittshälften 99 beim Endeffektor 14, mittels einer Verbindungseinrichtung 348 miteinander verbunden. Diese umfasst auf jeder Verbindungsfläche 400 der Abschnittshälften 399, die im zusammengefügten Zustand aneinander anliegen, ein erstes Verbindungselement 352 in Form eines sich parallel zur Längsachse 324 erstreckenden Vorsprungs, welcher in Richtung auf die Verbindungsfläche der jeweils anderen Abschnittshälfte 399 vorsteht. Parallel zum Verbindungselement 352 ist ein weiteres Verbindungselement 356 in jeder Abschnittshälfte 399 ausgebildet, und zwar in Form einer zum Vorsprung korrespondierenden Ausnehmung. Werden die Abschnittshälften 399 miteinander verbunden, greift jeweils ein Verbindungselement 352 formschlüssig in das andere Verbindungselement 356 der jeweils anderen Abschnittshälfte 399 ein, so dass insgesamt der zylindrische Kraftübertragungsabschnitt 349 ausgebildet wird.

Von jeder Abschnittshälfte 399 erstreckt sich jeweils ein schmaler Steg in distaler Richtung weisend, und zwar vom Endeffektorteil 220 ein Steg 394 und vom Endeffektorteil 322 ein Steg 380. Die Stege 394 und 380 bilden jeweils streifenförmige Anlenkglieder 395 beziehungsweise 381 zur Ausbildung von Festkörpergelenken 396 beziehungsweise 382. Die Stege 394 und 380 gehen jeweils in einen Zugabschnitt 332 über, die bei beiden Endeffektorteilen 320 und 322 identisch ausgebildet sind. Die in etwa halbzylindrischen Zugabschnitte 332 mit den daran angeformten Werkzeugenden 390 können ineinander überführt werden durch Rotation um die Längsachse 324 um 180°. Die Werkzeugenden 390 sind an den Zugabschnitten 332 so angeordnet, dass sie in etwa parallel zueinander angeordnet sind und für Schneideinrichtungen in übliche Weise aneinander abgleitende Schneidkanten 370 definieren.

Zur verschwenkbaren Lagerung der Werkzeugelemente 318 auf einer nicht dargestellten, an einem Schaft montierbaren und eine Schwenkachse 316 quer zur Längsachse 324 definierenden Lagerwelle sind durch einen sich parallel zum Steg 394 erstreckenden Spalt 392 zwei Lagerbacken 387 ausgebildet, die in Richtung der Schwenkachse 316 voneinander durch einen Spalt 334 beabstandet sind. Proximalseitig sind die Lagerbacken 387 wie die Lagerbacken 287 durch einen Verbindungssteg 342 miteinander verbunden.

Am Zugabschnitt 332 des Endeffektorteils 322 ist ein einzelner Lagerbacken 385 in proximaler Richtung abstehend angeordnet und durch einen parallel zum Steg 380 verlaufenden Spalt 362 getrennt. Eine Breite des Lagerbackens 385 entspricht einer Breite des Spalts 334, so dass der Lagerbacken 385 zwischen die Lagerbacken 387 eingreifen kann. Die Lagerbacken 385 und 387 sind jeweils mit koaxial zur Schwenkachse 316 ausgerichteten Bohrungen 383 beziehungsweise 388 versehen.

Bis auf die Ausbildung der jeweiligen Lagerbacken 385 beziehungsweise 387 sind die Endeffektorteile 320 und 322 identisch ausgebildet und können durch Rotation um die Längsachse 324 um 180° ineinander überführt werden. Die Anordnung der Lagerbacken 385 und 387 ist dagegen spiegelsymmetrisch zu einer die Schwenkachse 316 senkrecht schneidenden und die Längsachse 324 enthaltenden Spiegelebene. Die Zugabschnitte 332 werden jeweils durch einen kurzen halbzylindrischen Abschnitt gebildet, wobei Schnittlinien aneinander anliegender Schnittflächen der Zugabschnitte in Innenflächen 344 der Werkzeugenden 390 übergehen, deren freie Kanten die Schneidkanten 370 bilden.

Zum Schneiden mit dem Endeffektor 314 muss der Kraftübertragungsabschnitt 349 in proximaler Richtung bewegt werden. Ein Schneidvorgang kann also durch Übertragen einer Zugkraft bewirkt werden.

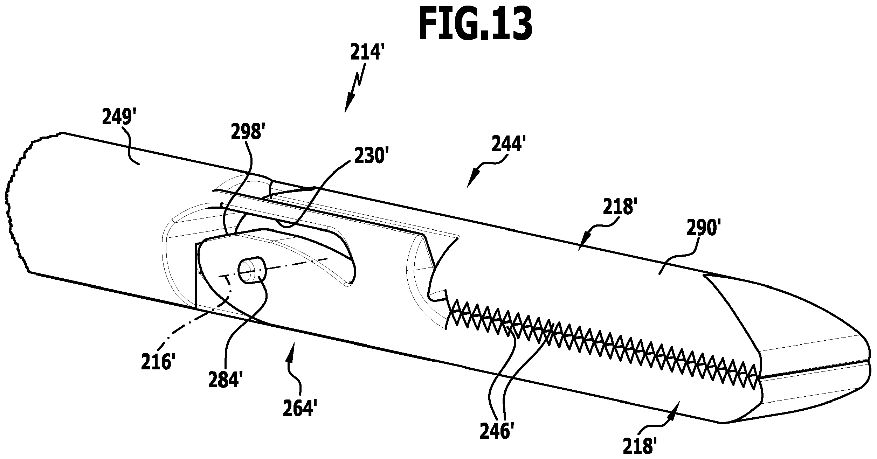

Ein weiteres Ausführungsbeispiel eines Endeffektors, welcher sowohl am Instrument 10 als auch am Instrument 120 vorgesehen sein kann, ist beispielhaft in den Figuren 13 bis 16 dargestellt und insgesamt mit dem Bezugszeichen 214' versehen. Der Endeffektor 214' entspricht in seinem prinzipiellen Aufbau dem im Zusammenhang mit den Figuren 6 bis 9 beschriebenen Endeffektor 214. Gleiche Teile sind daher der Übersichtlichkeit wegen mit denselben Bezugszeichen unter Hinzufügung eines ' versehen.

Der Endeffektor 214' umfasst zwei, um eine von einer an einem Schaft gehaltenen Lagerwelle 284' definierten Schwenkachse 216' gegeneinander verschwenkbar gelagerte Werkzeugelemente 218'. Insgesamt ist der Endeffektor 214' zweiteilig ausgebildet und umfasst einen ersten Endeffektorteil 220' und einen zweiten Endeffektorteil 222'. Die beiden Endeffektorteile 220' und 222' sind, anders als die Endeffektorteile 102, nicht identisch ausgebildet. Vielmehr ist der Endeffektorteil für sich betrachtet, ebenfalls anders als die Endeffektorteile 102, symmetrisch zu einer Spiegelebene ausgebildet, welche senkrecht zur Schwenkachse 216' verläuft und die Längsachse 224' des Endeffektors 214' enthält.

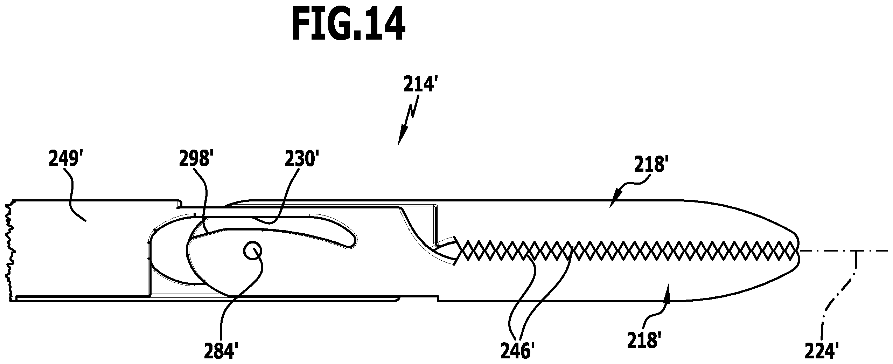

Der Endeffektorteil 220' umfasst einen zylindrischen Kraftübertragungsabschnitt 249', welcher auch als Kolben bezeichnet werden oder ein solcher sein kann. Ein proximales Ende desselben ist analog dem Kolben 48 geformt zur Verwendung in einem Instrument 10 oder ist mit einer Schub- und Zugstange 136 wie beim Instrument 120 verbindbar. Der Kraftübertragungsabschnitt 249' definiert eine in distaler Richtung weisende Stirnfläche 226'. Vom Kraftübertragungsabschnitt 249' stehen in distaler Richtung zwei parallel zueinander und zur Längsachse 224' sowie voneinander beabstandet verlaufende Stege 294' ab, welche flexible Anlenkglieder 295' zur Ausbildung eines Festkörpergelenks 296' definieren. Die Anlenkglieder 295' verlaufen zudem parallel zur Längsachse 224', wenn die Werkzeugelemente 218' eine beispielsweise in den Figuren 13 und 14 dargestellte Grundstellung einnehmen, in welcher die Anlenkglieder 295' nicht verformt sind und die Klemmflächen 244' der Werkzeugelemente 218' aneinander anliegen. Eine von der Längsachse 224' weg weisende Außenfläche 228' der Stege 294' bildet eine Fortsetzung einer zylindrischen Außenfläche des Kraftübertragungsabschnitts 249'. Eine in Richtung auf die Längsachse 224' hin weisende Innenfläche 230' des jeweiligen Stegs 294' ist zumindest abschnittsweise eben.

Das Anlenkglied 295' ist an einen zylindrischen Zugabschnitt 232' des Werkzeugelements 218' angeformt. Ein sich parallel zu den Stegen 294' erstreckender Spalt 292' trennt diese von zwei Lagerbacken 287', die wiederum durch einen Spalt 234' voneinander beabstandet sind. Die Lagerbacken 287' weisen in proximaler Richtung und sind koaxial zur Schwenkachse 216' mit einer Bohrung 283' versehen, durch die die stiftförmige Lagerwelle 284' durchgesteckt werden kann, um das Werkzeugelement 218' des Endeffektorteils 220' verschwenkbar an einem Schaft zu lagern. Etwas distalseitig eines Verbindungsbereichs 236' zwischen den Stegen 294' und dem Zugabschnitt 232' ist der Zugabschnitt 232' mit einer Durchbrechung 238' versehen, welche symmetrisch zur Symmetrieebene und quer zur Längsachse 224' den Zugabschnitt 232' in etwa quaderförmig durchbricht und eine in distaler Richtung weisende, schwach konkav gekrümmte Stirnfläche 240' des Zugabschnitts 232 eröffnet. Der Spalt 234' und die Durchbrechung 238' sind miteinander verbunden. Vom Zugabschnitt 232' steht in distaler Richtung weisend ein Werkzeugende 290' ab, und zwar derart, dass eine virtuelle Verlängerung derselben in proximaler Richtung den Stegen 294' diametral gegenüberliegen würde. Eine in Richtung auf die Längsachse 224' hin weisende Klemmfläche 244' ist mit einer Querverzahnung 246' versehen, welche eine Höhe in einem Bereich zwischen 20 µm und 100 µm aufweisen kann.

Zum Verbinden des Endeffektorteils 220' mit dem Endeffektorteil 222' dient eine Verbindungseinrichtung 248' mit einem ersten Verbindungselement 252', welches am Kraftübertragungsabschnitt 249' ausgebildet ist, und zwar in Form einer quaderförmigen Ausnehmung, die in einer von der Längsachse 224' weg weisenden radialen Richtung geöffnet ist. Von einem Boden 253' der Ausnehmung steht eine langgestreckte quaderförmige Leiste 254' ab, die in einer Richtung parallel zur Längsachse 224' etwas kürzer ist als die Ausnehmung, jedoch bündig mit der Stirnfläche 226' abschließt. Eine Breite quer zur Längsachse 224' des Verbindungselements 252' entspricht einer Breite der Durchbrechung 238' sowie des Spalts 234'.

Die Verbindungseinrichtung 248' umfasst ein zweites Verbindungselement 256', welches derart geformt ist, dass es die vom Verbindungselement 252' definierte Ausnehmung vollständig ausfüllt. Es ist daher insgesamt etwa quaderförmig und weist einen äußeren Oberflächenabschnitt auf, welcher den Kraftübertragungsabschnitt 249' zum Zylinder ergänzt. Zur Aufnahme der Leiste 254' ist eine Nut 258' im Verbindungselement 256' ausgebildet, welche proximalseitig verschlossen ist. So kann die Leiste 254' in die Nut 258' eingreifen, eine Relativbewegung der beiden Verbindungselemente 252' und 256' relativ zueinander parallel zur Längsachse 224' wird jedoch verhindert.

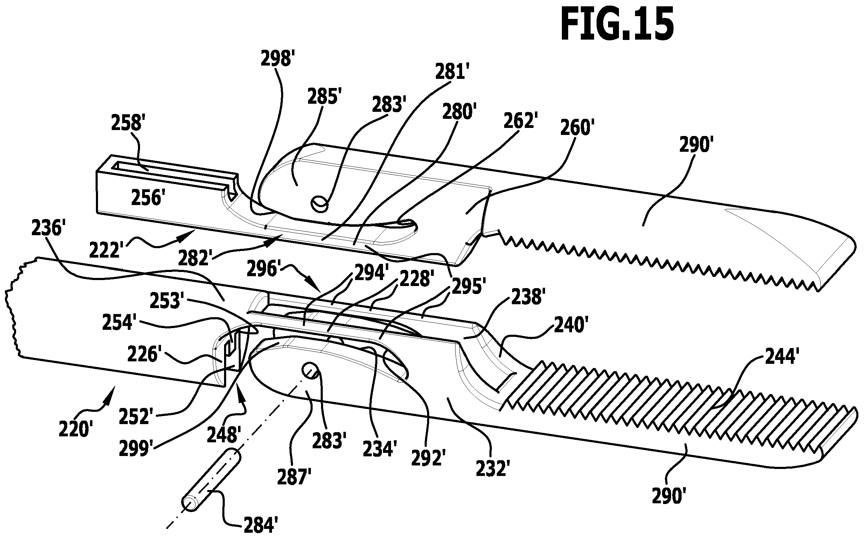

Den Stegen 294' diametral gegenüberliegend erstreckt sich vom Verbindungselement 256' ein Steg 280' in distaler Richtung, welcher ein Anlenkglied 281' definiert, welches ein Festkörpergelenk 282' zum Übertragen von Schub- und Zugkräften vom Kraftübertragungsabschnitt 249' auf das vom zweiten Endeffektorteil 222' umfasste Werkzeugelement 218' bildet. Der Steg 280' endet an einem backenförmigen Zugabschnitt 260', von dem in proximaler Richtung und schräg auf die Längsachse 224' hin weisend ein Lagerbacken 285' absteht, welcher mit einer Bohrung 283' versehen ist. Der Lagerbacken 285' ist durch einen kurzen, sich in distaler Richtung etwas über die Schwenkachse 216' hinaus erstreckenden Spalt 262' vom Steg 280' getrennt. Der Zugabschnitt 260', der Steg 280' und der Lagerbacken 285' sind alle in etwa gleich breit, wobei ihre Breite einer Breite des Verbindungselements 256' und damit einer Breite der Durchbrechung 238' entspricht. Dies ermöglicht es, zu einer Montage des Endeffektors 214' die getrennt voneinander gefertigten Endeffektorteile 220' und 222' derart zusammenzubauen, dass die, wie in Figur 15 dargestellten, voneinander getrennten Endeffektorteile 220' und 222' durch Einführen des Verbindungselements 256' in die Durchbrechung 238' von oben her kommend, das heißt in Richtung auf die Klemmfläche 244' und die Längsachse 224' hin weisend, das Verbindungselement 256' durch die Durchbrechung 238' durchgeschoben werden kann, bis es auf der anderen Seite hervorsteht. Dann werden die Endeffektorteile 220' und 222' relativ zueinander derart verschwenkt, dass das Verbindungselement 256' in das Verbindungselement 252' eingreifen kann. Die Bohrungen 283' und 288' sind fluchtend ausgerichtet und ein sich vom Zugabschnitt 260' in distaler Richtung erstreckendes Werkzeugende 290', welches dann eine Grundstellung einnimmt, wie sie in den Figuren 13 und 14 dargestellt ist, ist symmetrisch und dem anderen Werkzeugende 290' gegenüberliegend angeordnet. Der Endeffektor 214' kann dann mit der Lagerwelle 284' an einem Schaft des Instruments festgelegt werden. Optional können die Endeffektorteile 220' und 222' im Bereich der Verbindungseinrichtung 248' verklebt und/oder verschweißt werden. Wird der Kraftübertragungsabschnitt 249' in einem hülsenförmigen Schaft geführt, verhindert dieser eine Trennung der beiden Teile, so dass eine Verklebung oder Verschweißung nicht zwingend erforderlich ist.

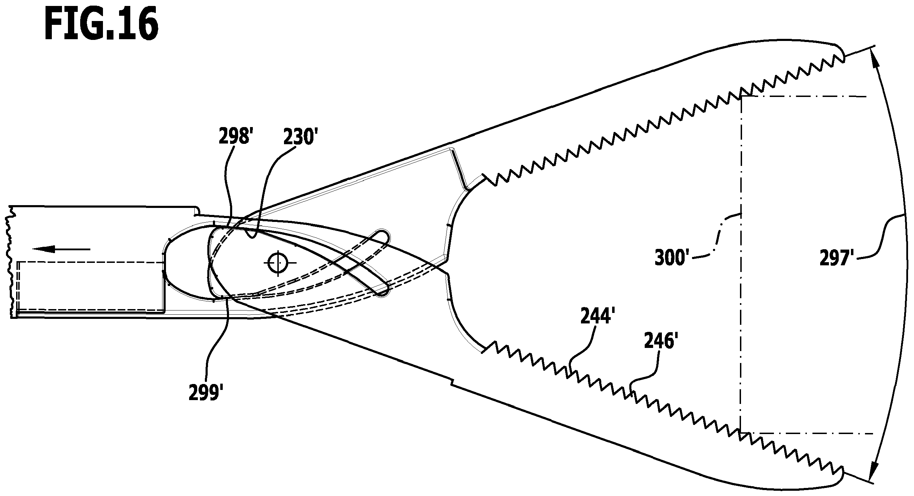

Der Endeffektor 214' ist aufgrund der durchdringenden oder durchgreifenden Anordnung der Werkzeugelemente 218' derart ausgebildet, dass eine von einem Kraftübertragungsglied eingeleitete Schubkraft zu einer Öffnungsbewegung des Endeffektors 214' führt, das heißt die Klemmflächen 244 werden voneinander weg verschwenkt. Die Anlenkglieder 281' und 295' werden dabei in Richtung auf die Schwenkachse 216' hin verbogen. Ab einem bestimmten Öffnungswinkel 297' der Werkzeugelemente 218' können die Lagerbacken 285' und 287' die Anlenkglieder 281' und 295' berühren. An den Lagerbacken 285' und 287' sind im Bereich von deren proximalen Enden kleine ebene, flächige viereckige Anlenkgliedstützabschnitte 298' und 299' ausgebildet, welche in der in den Figuren 13 und 14 dargestellten Grundstellung im Wesentlichen in Richtung auf die Anlenkglieder 281' beziehungsweise 295' hin weisen, jedoch von diesen beabstandet sind. Wird ein bestimmter Öffnungswinkel 297' überschritten, liegen die Anlenkgliedstützabschnitte 298' und 299' flächig an den Anlenkgliedern 281' beziehungsweise 295' an, wie dies in Figur 16 beispielhaft dargestellt ist. Werden die beiden Werkzeugelemente 218' gegeneinander blockiert, beispielsweise durch einen zwischen den Klemmflächen 244' klemmend gehaltenen Gegenstand 300', bei dem es sich beispielsweise um Gewebe oder ein Instrumententeil handeln kann, so können die Werkzeugelemente 218' nicht weiter aufeinander zu bewegt werden, auch dann, wenn der Kraftübertragungsabschnitt 249' auf Zug belastet wird. Allerdings würde die Einleitung von Zugkräften zu einem sogenannten "Straffziehen" der Festkörpergelenke 282' und 296' führen, wodurch hohe Kerbspannungen in den Anfangs- und Endbereichen der Festkörpergelenk 282' und 296' auftreten können. Durch das Abstützen der Anlenkglieder 281' und 295' mittels der Anlenkgliedstützabschnitte 298' und 299' der Lagerbacken 285' und 287' können solche Kerbspannungen reduziert werden, ebenso wie eine Bauteilbelastung im belasteten Zustand der Werkzeugelemente 218' unter großen Öffnungswinkeln 297', wie beispielsweise in Figur 16 dargestellt. Durch das Abstützen wird ferner die Steifigkeit des Endeffektors 214' bei Greifvorgängen erhöht. Der beschriebene Abstützungseffekt tritt abhängig von der jeweiligen Belastungssituation, bereits bei Öffnungswinkeln 297' der Werkzeugelemente 218' unterhalb eines maximalen Zielöffnungswinkels ein, das heißt Abstützungseffekte, wie beschrieben, sind an den Festkörpergelenken 282' und 296' bei Greifvorgängen unter großen Öffnungswinkeln 297' zu beobachten. Die besonderen Abstützeigenschaften werden insbesondere dadurch erreicht, dass die Anlenkgliedstützabschnitte 298' und 299' proximalseitig der Schwenkachse 216' ausgebildet sind.

Die Endeffektoren 14, 214, 214' und 314, insbesondere deren Endeffektorteile 102, 220 und 222, 220' und 222' sowie 320 und 322 können sowohl aus einem Metall, insbesondere einem Instrumentenstahl, oder einem Kunststoff hergestellt sein. Für Metalle eignen sich insbesondere Bearbeitungsverfahren wie Schleifen, Fräsen, Drahterodieren oder Laserschneiden. Für die Bearbeitung von Kunststoffen sind insbesondere Thermoformungsverfahren anwendbar, wie zum Beispiel Tiefziehen, Heißblasen, Prägen oder Spritzgießen. Als Kunststoffmaterialien eignen sich insbesondere Polyetheretherketon (PEEK), Polyethylen (PE), Polyurethan (PU), Polyethylenterephtalat (PET), Polycarbonat (PC) oder Polypropylen (PP). Insbesondere aus Kunststoffen hergestellte Endeffektoren ermöglichen es, den Schaft und den Endeffektor zusammen kostengünstig als Einwegbauteil herzustellen, welches nach einem chirurgischen Eingriff entsorgt werden kann. Insbesondere aufgrund der bei den beschriebenen Endeffektoren möglichen kleinen Abmessungen, es sind insbesondere Außendurchmesser kleiner als 2 mm möglich, ist eine Wiederaufbereitung der Endeffektoren nicht einfach und unter Umständen mit dem Risiko behaftet, dass keine Keimfreiheit gewährleistet werden kann.

Die beschriebenen Endeffektoren stellen lediglich Beispiele für Endeffektoren dar, die auf einer Lagerwelle schwenkbar gelagerte Werkzeugelemente umfassen, die über ein flexibles Anlenkglied in Form eines Festkörpergelenks von einem Kraftübertragungsglied mit einer Schub- und/oder Zugkraft beaufschlagt werden können.

Eine Rückstellung von einer ausgelenkten Werkzeugelementstellung der Werkzeugelemente in eine Grundstellung zurück, in welcher die jeweiligen Festkörpergelenke 96, 282, 296, 282', 296' und 396 unverformt sind, kann mittels einer Rückstelleinrichtung 164, 264, 264' beziehungsweise 364 erfolgen. Diese umfasst jeweils ein Rückstellglied in Form der flexiblen Stege 94, 280 und 294, 280' und 294' beziehungsweise 380 und 394. Aufgrund einer Verformung der Stege gespeicherte Energie kann zum Zurückführen der Werkzeugelemente in ihre Grundstellung genutzt werden. Daher sind die beschriebenen Endeffektoren auch optional derart nutzbar, dass zu einem Betätigen derselben stets nur Zug- oder Schubkräfte eingeleitet werden müssen.

## Patentansprüche

1. Chirurgisches Instrument (10; 120) mit einem Schaft (12; 122), einem mindestens ein beweglich gelagertes Werkzeugelement (18; 218; 318) umfassenden, eine Längsachse (24; 124; 224; 324) definierenden Endeffektor (14; 214; 314) und einem im Schaft (12; 122) in distaler und proximaler Richtung bewegbar gelagerten Kraftübertragungsglied (22; 136) zum Übertragen einer an einem proximalen Ende des Instruments (12; 120) eingeleiteten Betätigungskraft auf den Endeffektor (14; 214; 314) zum Bewegen des mindestens einen Werkzeugelements (18; 218; 318) von einer ersten in eine zweite Werkzeugelementstellung und/oder umgekehrt, wobei das mindestens eine Werkzeugelement (18; 218; 318) auf einer am Schaft (12; 122) gehaltenen Lagerwelle (84; 184) verschwenkbar gelagert ist, wobei das mindestens eine Werkzeugelement (18; 218; 318) über mindestens ein mindestens abschnittsweise flexibles Anlenkglied (95; 295; 395) mit dem Kraftübertragungsglied (22; 136) verbunden ist zum Übertragen der Betätigungskraft vom Kraftübertragungsglied (22; 136) auf das mindestens eine Werkzeugelement (18; 218; 318), **dadurch gekennzeichnet, dass** das mindestens eine Anlenkglied (95; 295; 395) unlösbar mit dem mindestens einen Werkzeugelement (18; 218; 318) verbunden ist.

2. Chirurgisches Instrument nach Anspruch 1, **dadurch gekennzeichnet, dass** das mindestens eine Anlenkglied (95; 295; 395) einstückig mit dem mindestens einen Werkzeugelement (18; 218; 318) ausgebildet ist.

3. Chirurgisches Instrument nach einem der voranstehenden Ansprüche, **dadurch gekennzeichnet, dass** das mindestens eine Anlenkglied (95; 295; 395) unlösbar mit dem Kraftübertragungsglied (22; 136) verbunden ist.

4. Chirurgisches Instrument nach einem der voranstehenden Ansprüche, **dadurch gekennzeichnet, dass** das mindestens eine Anlenkglied (95; 295; 395) ein Festkörpergelenk (96; 296; 396) zwischen dem Kraftübertragungsglied (22; 136) und dem mindestens einen Werkzeugelement (18; 218; 318) bildet.

5. Chirurgisches Instrument nach einem der voranstehenden Ansprüche, **dadurch gekennzeichnet, dass** das mindestens eine Anlenkglied (95; 295; 395) distalseitig der Lagerwelle (84; 184) am mindestens einen Werkzeugelement (18; 218; 318) angreift.

6. Chirurgisches Instrument nach einem der voranstehenden Ansprüche, **dadurch gekennzeichnet, dass** der Endeffektor (14; 214; 314) einen in distaler und proximaler Richtung bewegbaren Kraftübertragungsabschnitt (49; 249; 349) umfasst, welcher mit dem Kraftübertragungsglied (22; 136) verbunden ist, oder an diesem anliegt, und einen distalen Endbereich des Kraftübertragungsglieds (22; 136) bildet, und dass ein proximales Ende des mindestens einen Anlenkgliedes (95; 295; 395) mit dem Kraftübertragungsabschnitt (49; 249; 349) verbunden ist.

7. Chirurgisches Instrument nach einem der voranstehenden Ansprüche, **dadurch gekennzeichnet, dass** das Kraftübertragungsglied (22) mindestens einen im Schaft (12) verschiebbar gelagerten Kolben (36, 48) und einen vom mindestens einen Kolben (36, 48) durch ein Dichtungselement (68) getrennten Fluidabschnitt (60) umfasst, welcher mit einem Fluid (80) befüllt ist, welches durch Druckbeaufschlagung gegen das Dichtungselement (68) drückbar ist zum Bewegen des am Dichtungselement (68) anliegenden mindestens einen Kolbens (36, 48) parallel zur Längsachse (24) des Schafts (12).

8. Chirurgisches Instrument nach einem der voranstehenden Ansprüche, **gekennzeichnet durch** eine Führungseinrichtung zum Führen einer Bewegung des Kraftübertragungsabschnitts (49; 249; 349) und/oder des Kraftübertragungsglieds (22; 136) im Schaft (12; 122) parallel zur Längsachse (24; 124).

9. Chirurgisches Instrument nach einem der voranstehenden Ansprüche, **dadurch gekennzeichnet, dass** das mindestens eine Anlenkglied (95; 295; 395) in einer beliebigen von einer Grundstellung, in welcher es unverformt ist, abweichenden Arbeitsstellung des mindestens einen Werkzeugelements (18; 218; 318) verformt ist.

10. Chirurgisches Instrument nach einem der voranstehenden Ansprüche, **dadurch gekennzeichnet, dass** sich das mindestens eine Anlenkglied (95; 295; 395) parallel oder im Wesentlichen parallel zur Längsachse (24; 124; 224; 324) sowohl proximalseitig als auch distalseitig der Lagerwelle (84; 184) erstreckt.

11. Chirurgisches Instrument nach einem der voranstehenden Ansprüche, **dadurch gekennzeichnet, dass** das Anlenkglied (95; 295; 395) eine Dicke in einem Bereich von etwa 50 µm bis 700 µm aufweist.

12. Chirurgisches Instrument nach einem der voranstehenden Ansprüche, **dadurch gekennzeichnet, dass** eine Rückstelleinrichtung (164; 264; 364) vorgesehen ist zum Überführen des mindestens einen Werkzeugelements (18; 218; 318) aus einer beliebigen ausgelenkten Werkzeugelementstellung zurück in eine Grundstellung.

13. Chirurgisches Instrument nach einem der voranstehenden Ansprüche, **dadurch gekennzeichnet, dass** der Endeffektor (14; 214; 314) zweiteilig ausgebildet ist und zwei Endeffektorteile (102; 220, 222; 320, 322) umfasst und dass der Endeffektor (14) aus zwei identischen, fest miteinander verbundenen Endeffektorteilen (102) gebildet ist, welche relativ zueinander um 180° um die Längsachse (24) verdreht angeordnet sind.

14. Chirurgisches Instrument nach einem der voranstehenden Ansprüche, **dadurch gekennzeichnet, dass** die zwei Werkzeugelemente (218; 318) einander gegenseitig durchdringend oder kreuzend ausgebildet sind.

15. Chirurgisches Instrument nach einem der voranstehenden Ansprüche, **dadurch gekennzeichnet, dass** der Endeffektor (214; 314) derart ausgebildet ist, dass das mindestes eine Werkzeugelement (218; 318) infolge einer Bewegung des Kraftübertragungsglieds (22; 136) in proximaler Richtung in Richtung auf die Längsachse (24; 124; 224; 324) hin verschwenkbar ist und umgekehrt.

16. Chirurgisches Instrument nach einem der voranstehenden Ansprüche, **dadurch gekennzeichnet, dass** der Endeffektor (14) derart ausgebildet ist, dass das mindestes eine Werkzeugelement (18) infolge einer Bewegung des Kraftübertragungsglieds (22; 136) in distaler Richtung in Richtung auf die Längsachse (24; 124) hin verschwenkbar ist und umgekehrt.

17. Chirurgisches Instrument nach einem der voranstehenden Ansprüche, **dadurch gekennzeichnet, dass** an einem proximalen Ende des mindestens einen Werkzeugelements (218') ein Anlenkgliedstützabschnitt (298', 299') ausgebildet ist, welcher in Richtung auf das mindestens eine Anlenkglied (281'; 295') hin weist.

18. Chirurgisches Instrument nach Anspruch 17, **dadurch gekennzeichnet, dass** das mindestens eine Anlenkglied (281'; 295') in einer Werkzeugelementstellung, welche eine Grundstellung definiert, unverformt und vom Anlenkgliedstützabschnitt (298', 299') beabstandet ist und/oder dass sich in einer Werkzeugelementstellung, welche eine Zugstellung definiert, der Anlenkgliedstützabschnitt (298', 299') flächig am mindestens einen Anlenkglied (281'; 295') abstützt, wobei in der Zugstellung das mindestens eine Werkzeugelement (218') ausgelenkt und in der ausgelenkten Stellung blockiert ist.

## Claims

1. Surgical instrument (10; 120) having a shaft (12; 122), an end effector (14; 214; 314) comprising at least one movably mounted tool element (18; 218; 318) and defining a longitudinal axis (24; 124; 224; 324), and a force transmitting member (22; 136) mounted in the shaft (12; 122) for movement in the distal and proximal directions for transmitting an actuating force introduced at a proximal end of the instrument (12; 120) onto the end effector (14; 214; 314) in order to move the at least one tool element (18; 218; 318) from a first tool element position to a second tool element position and/or vice versa, the at least one tool element (18; 218; 318) being pivotably mounted on a bearing shaft (84; 184) held on the shaft (12; 122), the at least one tool element (18; 218; 318) being connected by at least one articulation member (95; 295; 395), at least a section of which is flexible, to the force transmitting member (22; 136) in order to transmit the actuating force from the force transmitting member (22; 136) onto the at least one tool element (18; 218; 318), **characterized in that** the at least one articulation member (95; 295; 395) is undetachably connected to the at least one tool element (18; 218; 318).

2. Surgical instrument in accordance with claim 1, **characterized in that** the at least one articulation member (95; 295; 395) is formed in one piece with the at least one tool element (18; 218; 318).

3. Surgical instrument in accordance with any one of the preceding claims, **characterized in that** the at least one articulation member (95; 295; 395) is undetachably connected to the force transmitting member (22; 136).

4. Surgical instrument in accordance with any one of the preceding claims, **characterized in that** the at least one articulation member (95; 295; 395) forms a flexure hinge (96; 296; 396) between the force transmitting member (22; 136) and the at least one tool element (18; 218; 318).

5. Surgical instrument in accordance with any one of the preceding claims, **characterized in that** the at least one articulation member (95; 295; 395) engages the at least one tool element (18; 218; 318) distally of the bearing shaft (84; 184).

6. Surgical instrument in accordance with any one of the preceding claims, **characterized in that** the end effector (14; 214; 314) comprises a force transmitting section (49; 249; 349) movable in the distal and proximal directions, which is connected to or lies against the force transmitting member (22; 136) and forms a distal end area of the force transmitting member (22; 136), and **in that** a proximal end of the at least one articulation member (95; 295; 395) is connected to the force transmitting section (49; 249; 349).

7. Surgical instrument in accordance with any one of the preceding claims, **characterized in that** the force transmitting member (22) comprises at least one piston (36, 48) mounted for displacement in the shaft (12), and a fluid section (60) separated from the at least one piston (36, 48) by a sealing element (68) and filled with a fluid (80) which is pressable against the sealing element (68) by the application of pressure in order to move the at least one piston (36, 48) lying against the sealing element (68) parallel to the longitudinal axis (24) of the shaft (12).

8. Surgical instrument in accordance with any one of the preceding claims, **characterized by** a guide device for guiding a movement of the force transmitting section (49; 249; 349) and/or the force transmitting member (22; 136) in the shaft (12; 122) parallel to the longitudinal axis (24; 124).

9. Surgical instrument in accordance with any one of the preceding claims, **characterized in that** the at least one articulation member (95; 295; 395) is deformed in any operating position of the at least one tool element (18; 218; 318) that differs from a normal position in which it is undeformed.

10. Surgical instrument in accordance with any one of the preceding claims, **characterized in that** the at least one articulation member (95; 295; 395) extends parallel or substantially parallel to the longitudinal axis (24; 124; 224; 324) both proximally and distally of the bearing shaft (84; 184).

11. Surgical instrument in accordance with any one of the preceding claims, **characterized in that** the articulation member (95; 295; 395) has a thickness ranging from approximately 50 µm to 700 µm.

12. Surgical instrument in accordance with any one of the preceding claims, **characterized in that** a resetting device (164; 264; 364) is provided for transferring the at least one tool element (18; 218; 318) from any swivelled position of the tool element back into a normal position.

13. Surgical instrument in accordance with any one of the preceding claims, **characterized in that** the end effector (14; 214; 314) is of two-part construction and comprises two end effector parts (102; 220; 222; 320; 322), and **in that** the end effector (14) is formed by two identical end effector parts (102) fixedly connected to each other, which are arranged in a manner rotated through 180° about the longitudinal axis (24) relative to each other.

14. Surgical instrument in accordance with any one of the preceding claims, **characterized in that** the two tool elements (218; 318) are constructed so as to mutually penetrate or intersect each other.

15. Surgical instrument in accordance with any one of the preceding claims, **characterized in that** the end effector (214; 314) is so constructed that as a result of a movement of the force transmitting member (22; 136) in the proximal direction, the at least one tool element (218; 318) is pivotable towards the longitudinal axis (24; 124; 224; 324) and vice versa.

16. Surgical instrument in accordance with any one of the preceding claims, **characterized in that** the end effector (14) is so constructed that as a result of a movement of the force transmitting member (22; 136) in the distal direction, the at least one tool element (18) is pivotable towards the longitudinal axis (24; 124) and vice versa.

17. Surgical instrument in accordance with any one of the preceding claims, **characterized in that** an articulation member supporting section (298', 299') facing the at least one articulation member (281'; 295') is formed at a proximal end of the at least one tool element (218').

18. Surgical instrument in accordance with claim 17, **characterized in that** the at least one articulation member (281'; 295'), in a position of the tool element defining a normal position, is undeformed and spaced from the articulation member supporting section (298', 299'), and/or **in that** in a position of the tool element defining a pulled position, the articulation member supporting section (298', 299') is supported with surface-to-surface contact on the at least one articulation member (281'; 295'), and, in the pulled position, the at least one tool element (218') is swivelled out and blocked in the swivelled-out position.

## Revendications

1. Instrument chirurgical (10; 120) comprenant un corps allongé en forme de tige (12; 122), un effecteur terminal (14; 214; 314) définissant un axe longitudinal (24; 124; 224; 324) et comportant au moins un élément d'outil (18; 218; 318) monté de manière mobile, et un organe de transmission de force (22; 136), qui est monté de manière mobile en direction distale et proximale dans le corps allongé en forme de tige (12; 122), et destiné à transmettre une force d'actionnement appliquée à une extrémité proximale de l'instrument (12; 120) à l'effecteur terminal (14; 214; 314), en vue de déplacer ledit au moins un élément d'outil (18; 218; 318) d'une première à une deuxième position d'élément d'outil et/ou inversement, instrument,
dans lequel ledit au moins un élément d'outil (18; 218; 318) est monté pivotant sur un arbre de palier (84; 184) maintenu sur le corps allongé en forme de tige (12; 122), et
dans lequel ledit au moins un élément d'outil (18; 218; 318) est relié, par l'intermédiaire d'au moins un organe d'articulation (95; 295; 395) au moins flexible par secteurs, à l'organe de transmission de force (22; 136), en vue de la transmission de la force d'actionnement de l'organe de transmission de force (22; 136) au dit au moins un élément d'outil (18; 218; 318),
**caractérisé en ce que** ledit au moins un organe d'articulation (95; 295; 395) est relié de manière inamovible au dit au moins un élément d'outil (18; 218; 318).

2. Instrument chirurgical selon la revendication 1,
**caractérisé en ce que** ledit au moins un organe d'articulation (95; 295; 395) est réalisé d'un seul tenant avec ledit au moins un élément d'outil (18; 218; 318).

3. Instrument chirurgical selon l'une des revendications précédentes, **caractérisé en ce que** ledit au moins un organe d'articulation (95; 295; 395) est relié de manière inamovible à l'organe de transmission de force (22; 136).

4. Instrument chirurgical selon l'une des revendications précédentes, **caractérisé en ce que** ledit au moins un organe d'articulation (95; 295; 395) forme une articulation monolithique (96; 296; 396) entre l'organe de transmission de force (22; 136) et ledit au moins un élément d'outil (18; 218; 318).

5. Instrument chirurgical selon l'une des revendications précédentes, **caractérisé en ce que** ledit au moins un organe d'articulation (95; 295; 395) agit sur ledit au moins un élément d'outil (18; 218; 318) à distance distale de l'arbre de palier (84 ; 184).

6. Instrument chirurgical selon l'une des revendications précédentes, **caractérisé en ce que** l'effecteur terminal (14; 214; 314) comporte un tronçon de transmission de force (49; 249; 349) mobile dans la direction distale et proximale, qui est relié à l'organe de transmission de force (22; 136) ou s'appuie contre celui-ci, et forme une zone d'extrémité distale de l'organe de transmission de force (22; 136), et **en ce qu'**une extrémité proximale dudit au moins un organe d'articulation (95; 295; 395) est reliée au tronçon de transmission de force (49; 249; 349).

7. Instrument chirurgical selon l'une des revendications précédentes, **caractérisé en ce que** l'organe de transmission de force (22) comprend au moins un piston (36, 48) monté coulissant dans le corps allongé en forme de tige (12), et au moins un tronçon à fluide (60) séparé par un élément d'étanchéité (68) dudit au moins un piston (36, 48) et rempli d'un fluide (80), qui, par une sollicitation en pression, peut être compressé contre l'élément d'étanchéité (68) pour déplacer ledit au moins un piston (36, 48) en appui contre l'élément d'étanchéité (68), parallèlement à l'axe longitudinal (24) du corps allongé en forme de tige (12).

8. Instrument chirurgical selon l'une des revendications précédentes, **caractérisé par** un dispositif de guidage destiné à guider un mouvement du tronçon de transmission de force (49; 249; 349) et/ou de l'organe de transmission de force (22; 136) dans le corps allongé en forme de tige (12; 122), parallèlement à l'axe longitudinal (24; 124).

9. Instrument chirurgical selon l'une des revendications précédentes, **caractérisé en ce que** ledit au moins un organe d'articulation (95; 295; 395) est déformé dans une position de travail quelconque dudit au moins un élément d'outil (18; 218; 318), différente de la position de base, dans laquelle il est non déformé.

10. Instrument chirurgical selon l'une des revendications précédentes, **caractérisé en ce que** ledit au moins un organe d'articulation (95; 295; 395) s'étend parallèlement ou sensiblement de manière parallèle à l'axe longitudinal (24; 124; 224; 324) aussi bien du côté proximal que du côté distal de l'arbre de palier (84; 184).

11. Instrument chirurgical selon l'une des revendications précédentes, **caractérisé en ce que** l'organe d'articulation (95; 295; 395) présente une épaisseur se situant dans une plage d'environ 50 µm à 700 µm.

12. Instrument chirurgical selon l'une des revendications précédentes, **caractérisé en ce qu'**il est prévu un dispositif de rappel (164; 264; 364) pour transférer ledit au moins un élément d'outil (18; 218; 318) d'une position déviée d'élément d'outil, en retour à une position de base.

13. Instrument chirurgical selon l'une des revendications précédentes, **caractérisé en ce que** l'effecteur terminal (14; 214; 314) est d'une configuration en deux pièces et comprend deux pièces d'effecteur terminal (102; 220, 222; 320, 322), et **en ce que** l'effecteur terminal (14) est formé de deux pièces d'effecteur terminal (102) identiques, reliées de manière fixe l'une à l'autre, et qui sont agencées en étant tournées l'une par rapport à l'autre de 180° autour de l'axe longitudinal (24).

14. Instrument chirurgical selon l'une des revendications précédentes, **caractérisé en ce que** les deux éléments d'outil (218; 318) sont configurés de manière à se traverser ou se croiser réciproquement l'un l'autre.

15. Instrument chirurgical selon l'une des revendications précédentes, **caractérisé en ce que** l'effecteur terminal (214; 314) est conçu pour que ledit au moins un élément d'outil (218; 318), suite à un mouvement de l'organe de transmission de force (22; 136) en direction proximale, puisse pivoter en direction de l'axe longitudinal (24; 124; 224; 324) et inversement.

16. Instrument chirurgical selon l'une des revendications précédentes, **caractérisé en ce que** l'effecteur terminal (14) est conçu pour que ledit au moins un élément d'outil (18), suite à un mouvement de l'organe de transmission de force (22; 136) en direction distale, puisse pivoter en direction de l'axe longitudinal (24; 124; 224; 324) et inversement.

17. Instrument chirurgical selon l'une des revendications précédentes, **caractérisé en ce qu'**à une extrémité proximale dudit au moins un élément d'outil (218'), est formé un tronçon d'appui d'organe d'articulation (298', 299'), qui est dirigé en direction dudit au moins un organe d'articulation (281'; 295').

18. Instrument chirurgical selon la revendication 17,
**caractérisé en ce que** ledit au moins un organe d'articulation (281'; 295'), dans une position d'élément d'outil, qui définit une position de base, est non déformé et est espacé du tronçon d'appui d'organe d'articulation (298', 299'), et/ou **en ce que**, dans une position d'élément d'outil, qui définit une position de traction, le tronçon d'appui d'organe d'articulation (298', 299') s'appuie en surface sur ledit au moins un organe d'articulation (281'; 295'), ledit au moins un élément d'outil (218') étant dévié, dans la position de traction, et bloqué dans la position déviée.
